# EUROPEAN PATENT SPECIFICATION

(11) **EP 4 499 802 B1**
(45) Date of publication and mention of the grant of the patent: **11.06.2025**
(21) Application number: 23735981.5
(22) Date of filing: 22.06.2023
(51) Int. Cl.: C12M 3/00, C12M 1/00, C12M 1/02, C12M 1/34, F16L 37/35

(54) **A MODULAR INCUBATOR SYSTEM**
MODULARES INKUBATORSYSTEM
SYSTÈME D'INCUBATEUR MODULAIRE

(30) Priority: 07.11.2022 DK PA202201009
(43) Date of publication of application: 05.02.2025
(73) Proprietor: Esco Medical Technologies, UAB, 51230 Kaunas (LT)
(72) Inventor: NIELSEN, Ricky Lindgaard, 8250 Egå (DK); MIKKELSEN, Mikkel Hjerrild, 7100 Vejle (DK)
(74) Representative: Skov, Anders
(86) International application number: PCT/EP2023/066987
(87) International publication number: WO 2024/099598

(56) References cited:
- DE-U1- 20 011 308
- US-A1- 2010 316 446
- US-A1- 2018 023 149
- US-A1- 2022 081 670
- US-A1- 2022 341 853

## Description

### Field of the invention

The present invention relates in general to the field of incubation of viable biological materials and in particular to incubators for IVF (in vitro fertilization) procedures.

More specifically, the present invention relates in a first aspect to a modular incubator system for incubating a viable biological material, wherein the modular incubator system comprises one or more modular incubator chambers in combination with a docking station.

In a second aspect the present invention relates to a modular incubator chamber for incubating a viable biological material.

In a third aspect the present invention relates to a docking station for docking one or more modular incubator chambers.

In a fourth aspect the present invention provides a use of a modular incubator system according to the first aspect of the invention for incubating a viable biological material.

In a fifth aspect the present invention provides a use of a modular incubator chamber according to the second aspect of the invention for incubating a viable biological material.

In a sixth aspect the present invention provides a use of a docking station according to the third aspect of the invention for incubating a viable biological material.

In a seventh aspect the present invention provides a method for incubating a viable biological material by using a modular incubator system according to the first aspect of the present invention.

### Background of the invention

The development of in vitro fertilization (IVF) has for the latest few decades resulted in considerably improved methods and techniques which have enhanced success rates of IVF mediated pregnancies and births.

In vitro fertilization involves capturing a ripened egg from a female ovary, fertilizing the ovary with a spermatozoon, incubating the fertilized egg under a controlled environment and subsequently inserting the fertilized and incubated egg into a female's uterus.

As in vitro fertilization is most commonly used by females or couples which notoriously are having problems in getting pregnant the natural way, thus implying some degree of reduced fertility by the male or female counterpart of the couple, or both, and as in vitro fertilization techniques involves quite expensive procedures, these in vitro fertilization techniques are usually performed in a way that seek to optimize efficiency, especially in view of the fact that frequently more than one insertion of a fertilized egg into the female' uterus will be necessary in order to encounter a successful pregnancy.

Additionally, compared to the natural way of getting pregnant, in respect of a couple wherein an individual is having a generic disease or in case of suspicion of such, an IVF mediated pregnancy may be advantageous.

Accordingly, in order to make the in vitro fertilization techniques efficient, the female is typically provided with a hormone treatment prior to harvesting eggs from her ovary. Such hormone treatment will make the female ovary ovulate not only one egg, but a multitude of eggs at the same time.

In order to increase the chance of a viable and successful pregnancy more than one egg from the same female will accordingly be fertilized and incubated concurrently in an incubator.

Prior art incubators include a compartment which allows for accommodating more than one culture dish comprising the fertilized eggs.

Performing a successful in vitro fertilization and incubation of a fertilized egg is not an easy task. One of the major reasons for the rather low success rate of in vitro fertilizations is the absence of reliable methods for providing and maintaining optimum growth conditions for the embryo.

Some improved prior art incubators comprise a housing having one or more doors for providing access to the interior of the incubator. The interior of the incubator holds one or more culture dishes accommodating the embryos to be cultured. Such incubators may be provided with various regulation means for controlling humidity, temperature and gas composition of the interior of the incubator.

Recently, smaller modular incubators have been introduced in the marked. These modular incubators are configured to be stored in docking ports in a docking station which may provide the controlling of physical and chemical parameters encountered by the embryos being accommodated therein. Once any manual manipulation steps in respect of the embryo is needed, such as manual inspection or adding or removing or exchanging growth media, the modular incubator may be removed from the docking port of the docking station and arranged on a laboratory bench for easy access to the embryo.

In order to improve monitoring of the embryos being incubated in such a modular incubator chamber of a modular incubator system, one may envisage incorporating an image capturing device in the docking ports of the docking station. In this way it will be possible to provide optical monitoring of the embryos being accommodated in the modular incubator chambers and which are being incubated, provided that some type of transparent window is provided in the housing of the modular incubator chamber which allows the image capturing device to capture images through that transparent window.

It has been generally found that even very small deviations from what is considered to be optimum incubation conditions for an embryo being incubated in an incubator may have detrimental effects on the quality of the embryo being incubated and hence may reduce the success rate of a resulting pregnancy.

It has even been suggested that excessive exposure to light to an embryo being incubated in an IVF procedure may have a detrimental effect on a resulting pregnancy.

US2018/023149 discloses a modular incubator system suitable for incubating a viable biological material, such as oocytes, comprising one or more modular incubator chambers in combination with a docking station. The chamber comprises a transparent window for enabling capturing of images of a biological material accommodated in the internal volume of the chamber. The docking station comprises an image capturing device for capturing an image of the interior of the chamber, once being docking in a docking port.

Accordingly, simply providing a docking station of the prior art modular incubator systems with an image capturing device may provide benefits in terms of the ability to be able to visually monitor the morphological development of the embryo while being incubated; however, this will be at the cost of the detrimental effects of exposing the embryo to light through the transparent window in the modular incubator chamber, at least in the situations where the modular incubator chamber is being moved from its docking port of the docking station to the laboratory bench where manual manipulation steps are being performed.

Such detrimental effects may accordingly represent enhanced risks that the IVF procedure ends in an unsuccesful pregnancy once the embryo will been inserted into a female's uterus at a later stage in the process.

Accordingly, a need persists for improved modular incubators which allows for performing visual monitoring of an embryo, while incubating that embryo and without the detriment effects involved in excessively exposing the embryo to light.

It is an objective of the present invention to fulfill such need.

### Brief description of the invention

These objectives are fulfilled according to the various aspect of the present invention.

Accordingly, the first aspect of the present invention relates to a modular incubator system for incubating a viable bilogical material, said modular incubator system comprising:
- one or more modular incubator chambers in combination with
- a docking station;

wherein in respect of one or more of said one or more modulator incubator chambers, said modular incubator chamber comprises a housing having a first end and a second end, thereby defining a longitudinal direction X between said first end and said second end;
wherein said housing comprises a lid, wherein said lid is being configured to be able to shift between an open configuration allowing access to the interior of said modular incubator chamber and a closed configuration, sealing off access to the interior of said modular incubator chamber;
wherein said modular incubator chamber, at said interior thereof, comprises a culture dish support for positioning a culture dish with the view to accommodate one or more biological materials M within the housing of said modular incubator chamber;
wherein said housing of said modular incubator chamber comprises a transparent window for enabling capturing of images of a biological material M being accommodated in the interior thereof, through said transparent window;
wherein said housing of said modular incubator chamber comprises a shutter mechanism, wherein said shutter mechanism is configured to be able to shift between an open configuration and a closed configuration and vice versa;
wherein said shutter mechanism comprises a first shutter mechanism engagement means for enabling shifting said shutter mechanism between its open configuration and its closed configuration and vice versa, upon exerting a force thereto;
wherein said shutter mechanism is being arranged relative to said transparent window of said housing in such a way that in its open configuration, said shutter mechanism is enabling transmittal of light, from the outside, into the interior of said modular incubator chamber through said transparent window; and in such a way that in its closed configuration, said shutter mechanism is blocking transmittal of light, from the outside, into the interior of said modular incubator chamber through said transparent window;
wherein said docking station comprises one or more docking ports for receiving a housing of one or more of said incubator chambers;
wherein in respect of one or more docking ports of said docking station, said docking port comprises an image capturing device for capturing an image of the interior of a modular incubator chamber, once being docked in said docking port.

In a second aspect the present invention relates to a modular incubator chamber comprising:
a housing having a first end and a second end, thereby defining a longitudinal direction X between said first end and said second end;
wherein said housing comprises a lid, wherein said lid is being configured to be able to shift between an open configuration allowing access to the interior of said modular incubator chamber and a closed configuration, sealing off access to the interior of said modular incubator chamber;
wherein said modular incubator chamber, at said interior thereof, comprises a culture dish support for positioning a culture dish with the view to accommodate one or more biological materials M within the housing of said modular incubator chamber;
wherein said housing of said modular incubator chamber comprises a transparent window for enabling capturing of images of a biological material M being accommodated in the interior thereof, through said transparent window;
wherein said housing of said modular incubator chamber comprises a shutter mechanism, wherein said shutter mechanism is configured to be able to shift between an open configuration and a closed configuration and vice versa;
wherein said shutter mechanism comprises a first shutter mechanism engagement means for enabling shifting said shutter mechanism between its open configuration and its closed configuration and vice versa, upon exerting a force thereto;
wherein said shutter mechanism is being arranged relative to said transparent window of said housing in such a way that in its open configuration, said shutter mechanism is enabling transmittal of light, from the outside, into the interior of said modular incubator chamber through said transparent window; and in such a way that in its closed configuration, said shutter mechanism is blocking transmittal of light, from the outside, into the interior of said modular incubator chamber through said transparent window.

In a third aspect the present invention relates to a docking station for docking one or more modular incubator chambers; wherein said docking station comprises one or more docking ports for receiving a housing of one or more of said incubator chambers; wherein in respect of one or more docking ports of said docking station, said docking port comprises an image capturing device for capturing an image of the interior of a modular incubator chamber, once being docked in said docking port.

In a fourth aspect the present invention provides a use of a modular incubator system according to the first aspect of the present invention for incubating a viable biological material.

In a fifth aspect the present invention provides a use of a modular incubator chamber according to the second aspect of the present invention for incubating a viable biological material.

In a sixth aspect the present invention provides a use of a docking station according to according to the second aspect of the present invention for incubating a viable biological material.

In a seventh aspect the present invention provides method of incubating a viable biological material, wherein said method comprises:
i) providing a modular incubator system according to the first aspect of the present invention;
ii) providing a viable biological material;
iii) arranging said viable biological material in a culture dish and subsequently arranging said culture dish in the interior of a modular incubator chamber of said modular incubator system;
iv) docking said modular incubator chamber in a docking port of said docking station of said incubator system;
v) allowing said viable biological material to be incubated in said modular incubator chamber, while ensuring that said shutter mechanism of said modular incubator chamber is being in its closed configuration;
vi) whenever desired, shifting the configuration of said shutter mechanism of said modular incubator chamber to its open configuration and allowing said image capturing device to capture one or more images of said biological material being accommodated in said culture dish;
vii) optionally, upon finishing capture of images of said biological material, shifting the configuration of said shutter mechanism of said modular incubator chamber to its closed configuration

The present invention in its various aspects allows for enabling image capturing of a viable biological material, such as an oocyte or an embryo during incubation thereof, while at the same time minimizing any detrimental effects caused by exposure to the viable biological material for an excessive amount of light.

This will be so even if the modular incubator chamber is being removed from its respective docking port in the docking station.

### Brief description of the figures

Fig. 1 is a perspective view illustrating the general concept of designing an incubator as an incubator system comprising a plurality of modular incubator chambers in combination with a docking station having a plurality of docking ports..
Fig. 2a is a bottom perspective view showing part of a modular incubator chamber of the modular incubator system of the invention.
Fig. 2b is a partly exploded bottom perspective view showing the modular incubator chamber of Fig. 2a.
Fig. 2c is a is a partly exploded top perspective view showing the modular incubator chamber of Fig. 2b.
Fig. 3a is a cross-sectional view illustrating the the modular incubator chamber illustrated in Fig. 2a, 2b and 2c.
Fig. 3b is a cross-sectional view illustrating further details of the the modular incubator chamber of the present invention.
Fig. 4a and 4b are cross-sectional views illustrating details of a docking port of the docking station of the first aspect of the present invention.
Fig. 5a illustrates the shutter mechanism of a modular incubator chamber in its closed configuration in a bottom plan view (top drawing of Fig. 5a), in a cross-sectional view (middle drawing of Fig. 5a), and in a close-up cross-sectional view (bottom drawing of Fig. 5a).
Fig. 5b illustrates the shutter mechanism of a modular incubator chamber in its open configuration in a bottom plan view (top drawing of Fig. 5b), in a cross-sectional view (middle drawing of Fig. 5b), and in a close-up cross-sectional view (bottom drawing of Fig. 5b).
Fig. 6a is a front view looking into a docking port of a docking station of the modular incubator system of the present invention.
Fig. 6b is a perspective view of a modular incubator chamber seen from the first or rear end which is to enter a docking port of the modular incubator system of the present invention.
Fig. 7a and 7b are drawings illustrating the working modes of valves of a valve system to be used with the modular incubator chamber and the associated docking port of the docking station of the docking system of the present invention.
Fig. 8 is a diagram illustrating one embodiment of a design of a gas supply system comprising a gas source and a gas distribution system to be used with the docking station of the modular incubator system of the present invention.
Fig. 9 is a diagram illustrating the concept of gas source which may be incorporated in the docking station of the modular incubator system of the present invention.
Fig. 10 is a diagram illustrating the working mode of the controlling of the modular incubator system according to the invention.

### Detailed description of the invention

### The first aspect of the present invention

The fist aspect of the present invention relates to a modular incubator system 500 for incubating a viable biological material M, said modular incubator system comprising:
- one or more modular incubator chambers 300 in combination with
- a docking station 400;

wherein in respect of one or more of said one or more modular incubator chambers 300, said modular incubator chamber 300 comprises a housing 302 having a first end 340 and a second end 342, thereby defining a longitudinal direction X between said first end and said second end;
wherein said housing comprises a lid 304, wherein said lid is being configured to be able to shift between an open configuration allowing access to the interior 306 of said modular incubator chamber and a closed configuration, sealing off access to the interior of said modular incubator chamber;
wherein said modular incubator chamber 300, at said interior 306 thereof, comprises a culture dish support 308 for positioning a culture dish 310 with the view to accommodate one or more biological materials M within the housing 302 of said modular incubator chamber 300;
wherein said housing 302 of said modular incubator chamber 300 comprises a transparent window 316 for enabling capturing of images of a biological material M being accommodated in the interior thereof, through said transparent window;
wherein said housing 302 of said modular incubator chamber 300 comprises a shutter mechanism 344, wherein said shutter mechanism is configured to be able to shift between an open configuration and a closed configuration and vice versa;
wherein said shutter mechanism 344 comprises a first shutter mechanism engagement means 346 for enabling shifting said shutter mechanism between its open configuration and its closed configuration and vice versa, upon exerting a force thereto;
wherein said shutter mechanism 344 is being arranged relative to said transparent window 316 of said housing in such a way that in its open configuration, said shutter mechanism 344 is enabling transmittal of light, from the outside, into the interior of said modular incubator chamber through said transparent window 316; and in such a way that in its closed configuration, said shutter mechanism 344 is blocking transmittal of light, from the outside, into the interior of said modular incubator chamber through said transparent window 316;
wherein said docking station 400 comprises one or more docking ports 402 for receiving a housing 302 of one or more of said incubator chambers 300;
wherein in respect of one or more docking ports 402 of said docking station 400, said docking port comprises an image capturing device 408 for capturing an image of the interior 306 of a modular incubator chamber 300, once being docked in said docking port 402.

Accordingly, the present invention relates in its first aspect to a modular incubator system 500 comprising one or more modular incubator chambers 300 in combination with a docking station 400. The incubator chambers 300 and the docking station 400 are configured in such a way that the modular incubator chambers 300 can be docked in a docking port 402 and in such a way that an image capturing device 408 of the docking port will be able to capture images of a viable biological material being accommodated within the interior 306 of the modular incubator chamber 300 while being incubated therein and while the modular incubator chamber 300 is docked in that docking port 402.

The capturing of the images is performed through a transparent window 316 in the housing 302 of the modular incubator chamber 300. It is clear that such a transparent window will allow influx of electromagnetic radiation or light also in those periods of time where no image capturing is taking place.

In order to eliminate or at least considerably reduce the influx of electromagnetic radiation or light into the interior 306 of the housing 302 of the modular incubator chamber 300, and the detrimental effects associated therewith in respect of the viable biological material being incubated, the present invention provides a shutter mechanism 344, which is configured to be able to shift between an open configuration and a closed configuration and vice versa, and thereby allowing closing off influx of light in those periods of time where no image capturing is taking place.

Hereby, an environment which to a larger degree mimics the environment of a fallopian tube or a uterus of a female is obtained when a viable biological material is being incubated.

In the present invention the term "modular incubator system" shall be construed to mean a system comprising a docking station in combination with one or more incubator chambers, wherein the one or more incubator chambers is/are configured to be docked in respective docking ports of that docking station. The modular incubator system is intended for incubation or cultivation of a viable biological material.

The incubator system comprising the docking station and one or more incubator chamber(s) in general is configured for providing some kind of interaction between the docking station and the incubator chambers being docked therein.

Such interactions may be one or more of the following: providing a gas having a desired composition to the incubator chamber(s); providing electricity to the incubator chamber(s) for powering hearing elements thereof and/or for powering a light source in the incubator chamber(s); allowing monitoring of the viable biological material being present in the incubator chamber(s), such as by means of an image capturing device which is located in the docking station.

In should be understood that within the meaning of the present application, the term "modular incubator system" shall be construed in such a way that the incubator chambers are configured to be used for incubation of a viable biological material, irrespective of whether the individual incubator chamber is being docked in a docking port of the docking station, or whether that incubator chamber is removed from the docking port of the docking station.

In this way, it is to be understood that cultivation or incubation of a viable biological material of the individual incubator chambers may take place and/or be continued even after that incubator chamber has been removed from its docking station and placed e.g. on a laboratory bench. Hereby manual manipulation operations, such as shift or control of culture or growth media, manual inspection by use of a laboratory microscope or the like can take place. Such operation are preferably carried out under a hood providing a desired gas atmosphere.

In preferred embodiment, and in order to make such manual manipulation operations practical conceivable, when the individual incubation chamber has been removed from a docking port, the incubation chamber is configured in a way that enables support on a planar, horizontal support surface. This may be attained by providing the bottom part of the incubator chamber with one or more supports or simply by making the bottom part of the incubator chamber comprise a flat surface.

In preferred embodiments the incubation chamber, in the orientation intended during use for incubation, is having its maximum dimension in a horizontal direction.

In this way the dimension of the incubation chamber in a horizontal direction is greater than the dimension in a vertical direction. Hereby, adequate stability is attended when the incubator chamber is used for incubation at a location outside a docking port of the docking station.

The individual incubator chambers may in embodiments comprise a display, such as an electronic display, for providing information relating to the identity of the viable biological material being accommodated in the incubator chamber.

It should be understood that in some embodiments the present invention does not relate to methods or uses which involve treatment of the human or animal body by surgery or diagnostic methods practiced on the human or animal body.

It should also be understood that that in other embodiments the present invention may relate to methods or uses which involve treatment of the human or animal body by surgery or diagnostic methods practiced on the human or animal body.

In an embodiment of the modular incubator system 500 according to the first aspect of the present invention and in respect of one or more of said one or more modular incubator chambers 300 and in respect of one or more of said one or more docking ports 402 of said docking station 400, the position of said transparent window 316 of said modular incubator chamber 300 is adapted to the position of said image capturing device 408 in said docking port 402 in a way that enables capturing of images by said image capturing device 408 through said transparent window 316 of said modular incubator chamber 300, once said modular incubator chamber 300 is being docked in said docking port 402.

Hereby is ensured that the image capturing device 408 will be able to capture images of the interior 306 of that modular incubator chamber 300 through that transparent window 316.

In an embodiment of the modular incubator system 500 according to the first aspect of the present invention and in respect of one or more of said modular incubator chambers 300 and/or in respect of one or more of said docking ports 402 of said docking station 400, said modular incubator system comprises a shutter actuator 150, such as an electric shutter actuator which, upon being provided with a signal thereto, such as an electric signal, is being configured to exert a force to said first shutter mechanism engagement means 346, in order to shift the configuration of said shutter mechanism 344 of said incubator chamber 300 between said open configuration and said closed configuration, and vice versa.

Hereby remote control of the shutter mechanism 344 is possible.

In an embodiment of the modular incubator system 500 according to the first aspect of the present invention and in respect of one or more of said one or more modular incubator chambers 300, said shutter actuator 150 is being arranged in said modular incubator chamber 300 of said modular incubator system; or wherein in respect of one or more of said one or more docking ports 402 of said docking station 400, said shutter actuator 150 is being arranged in said docking port 402 of said modular incubator system.

Depending of the specific design of the modular incubator system 500 with its modular incubator chambers 300 and its docking station 400, it may be advantageous either to arrange the shutter actuator 150 in the modular incubator chamber 300 or in the docking port 402 of the modular incubator system 500.

In an embodiment of the modular incubator system 500 according to the first aspect of the present invention and in respect of one or more docking ports 402 of said docking station 400, said shutter actuator 150 comprises a second shutter mechanism engagement means 152, which is configured to exert a force to said first shutter mechanism engagement means 346 of said modular incubator chamber 300, thereby enabling shifting said shutter mechanism 344 between its open configuration and its closed configuration and vice versa, in a situation wherein said modular incubator chamber 300 is being docked in said docking port 402 of said docking station 400.

Hereby a force can be exerted to the first shutter mechanism engagement means 346 and thereby effect the shifting of the shutter mechanism 344 between its open configuration and its closed configuration and vice versa.

In an embodiment of the modular incubator system 500 according to the first aspect of the present invention and in respect of one or more of said one or more modular incubator chambers 300, said transparent window 316 of said modular incubator chamber 300 is arranged at a bottom part 358 of said housing 302 and wherein said shutter mechanism 344 is being arranged at said bottom part 358 of said housing 302, wherein said shutter mechanism 344 is being arranged below or above said transparent window 316.

As it is preferred to arrange the image capturing device 408 at a position in the docking port 402 of the docking station where it will focus in an upward direction, the transparent window 316 of the modular incubator chamber 300 is conveniently arranged at a bottom part 358 of the housing 302 of said modular incubator chamber 300.

In an embodiment of the modular incubator system 500 according to the first aspect of the present invention and in respect of one or more of said one or more modular incubator chambers 300, said culture dish support 308 of said modular incubator chamber 300 is being arranged above said transparent window 316.

Hereby is assured that when the image capturing device 408 focuses in a focus direction corresponding to said transparent window 316, that image capturing device 408 will focus to the area of a culture dish 410 being arranged on said culture dish support 308.

In an embodiment of the modular incubator system 500 according to the first aspect of the present invention and in respect of one or more of said one or more modular incubator chambers 300, said shutter mechanism 344 of said modular incubator chamber 300 comprises a plate shaped shutter element 360, wherein said plate shaped shutter element 360 is being arranged at said housing 302 of said modular incubator chamber 300 in such a way that said plate shaped element 360 is being able to be displaced relative to said housing 302, in a displacement direction D, between two positions, wherein in the first position 362 said shutter mechanism 344 is being in its closed configuration; and wherein in the second position 364, said shutter mechanism 344 is being in its open configuration.

In an embodiment and in said first position 362, said plate shaped shutter element 360 is being positioned relative to said transparent window 316 of said housing 302 so as to cover said transparent window 316, thereby blocking transmittal of light, from outside said housing 302 of said modular incubator chamber into the interior 306 of said housing through said transparent window 316 of said housing 302; and wherein in said second position 364, said plate shaped shutter element 360 is being positioned relative to said transparent window 316 of said housing 302 so as not to cover said transparent window 316, thereby enabling transmittal of light, from outside said housing 302 of said modular incubator chamber 300 into the interior 306 of said housing 302 through said transparent window of said housing,

In an embodiment said plate shaped shutter element 360 is having a through-going opening 366 therein, wherein in its first position 362, said through-going opening 366 of the plate shaped shutter element 360 of the shutter mechanism 344 is being positioned offset in relation to said transparent window 316 of said housing 302 of said modular incubator chamber 300, thereby blocking transmittal of light, from outside said housing 302 of said modular incubator chamber 300 into the interior 306 of said housing through said through-going opening 366 of the plate shaped shutter element 360 and through said transparent window 316 of said housing 302; and wherein in said second position 364, said through-going opening of said plate shaped shutter element 360 of the shutter mechanism 344 is being aligned with said transparent window 316 of said housing 302 of said modular incubator chamber 300, thereby enabling transmittal of light, from outside of said housing 302 of said modular incubator chamber 300 into the interior 306 of said housing through said through-going opening 366 of the plate shaped shutter element 360 and through said transparent window 316 of said housing 302.

Providing the shutter mechanism 344 with a plate shaped shutter element is a simple way of allowing the shutter mechanism 344 to shift between its open and closed configuration. And vice versa.

In an alternative embodiment the plate shaped shutter element 360 is having a rim 367, wherein in its first position 362, said rim 367 of the plate shaped shutter element 360 of the shutter mechanism 344 is being positioned on one side in relation to said transparent window 316 of said housing 302 of said modular incubator chamber 300, thereby blocking transmittal of light, from outside said housing 302 of said modular incubator chamber 300 into the interior 306 of said housing through said transparent window 316 of said housing 302; and wherein in said second position 364, said rim 367 of said plate shaped shutter element 360 of the shutter mechanism 344 is being positioned on the opposite side of said transparent window 316 of said housing 302 of said modular incubator chamber 300, thereby enabling transmittal of light, from outside said housing 302 of said modular incubator chamber 300 into the interior 306 of said housing 306 through said transparent window 316 of said housing 302 and by passing said rim 367 of the plate shaped shutter element 360.

In an embodiment said plate shaped shutter element 360 of said shutter mechanism 344 is being arranged at said housing 302, so as to be displaceable in a direction D which is parallel to said longitudinal direction X of said housing 302.

In an embodiment of the modular incubator system 500 according to the first aspect of the present invention and in respect of one or more of said one or more modular incubator chambers 300, said first shutter mechanism engagement means 346 of said shutter mechanism 344 comprises a shutter lever configured to be acted upon, by exerting a force to said shutter lever, thereby enabling shifting the configuration of said shutter mechanism between said open configuration and said closed configuration and vice versa.

In an embodiment the shutter lever is being connected to said plate shaped shutter element 360.

In an embodiment the second shutter mechanism engagement means 152 of said shutter actuator 150 is being configured to be in contact with said shutter lever of said first shutter mechanism engagement means 346, thereby exerting a force thereto, in the operation of shifting said shutter mechanism 344 between its open configuration and its closed configuration and vice versa.

Hereby the shutter mechanism 344 can be shifted between its open and closed configuration simply by exerting an external force to that shutter lever.

In an embodiment of the modular incubator system 500 according to the first aspect of the present invention and in respect of one or more of said one or more modular incubator chambers 300, said first shutter mechanism engagement means 346 of said shutter mechanism 344 comprises a first magnet 368 configured to be acted upon by a second magnet 450, thereby enabling shifting the configuration of said shutter mechanism 344 between said open configuration and said closed configuration, and vice versa, via movement of said second magnet 450.

This allows shifting the configuration of the shutter mechanism 344 between its open and closed configuration, and vice versa, via magnetic forces.

In an embodiment of the modular incubator system 500 according to the first aspect of the present invention and in respect of one or more of said one or more docking ports 402 of said modular incubator system, said docking port 402 comprises said shutter actuator 150, wherein said second shutter mechanism engagement means 152 of said shutter actuator comprises said second magnet 450 for exerting a magnetic force to said first magnet 368 of said first shutter mechanism engagement means 346 upon movement of said second magnet 450, thereby enabling shifting the configuration of said shutter mechanism 344, between said open configuration and said closed configuration and vice versa, by movement of said second magnet 450 via said shutter actuator 150.

This embodiment allows for shifting the configuration of the shutter mechanism 344 between its open and closed configuration, and vice versa, via a second shutter mechanism engagement means 152 of the shutter actuator which comprises a second magnet 450.

In an embodiment said first magnet 368 is being connected to said plate shaped shutter element 360 of said shutter mechanism 344.

In alternative embodiment the first magnet 368 is being replaced with a block of ferromagnetic material, such as iron or an iron alloy, or wherein said second magnet 450 is being replaced with a block of ferromagnetic material, such as iron or an iron alloy.

As iron and iron alloys are attached by magnets, one may use one magnet and one block of a ferromagnetic material instead of two magnets.

In an embodiment of the modular incubator system 500 according to the first aspect of the present invention the shutter actuator 150 is being an electric actuator.

Using an electric actuator enables remote control of the shutter mechanism 344 in a simple and easy way.

In an embodiment of the modular incubator system 500 according to the first aspect of the present invention and in respect of one or more docking ports 402 of said docking station 400, said docking port comprises said shutter actuator 150, wherein said shutter actuator comprises an electromotor 451 comprising a treaded rotatable axle 452, wherein said shutter actuator 150 comprises a treaded displacement element 454, wherein the threads of said axle 452 is being in engagement with the treads of said displacement element 454, and wherein said second shutter mechanism engagement means 152 is being connected to said threaded displacement element 454 by a connecting element 456, thereby enabling displacement of said second shutter mechanism engagement means 152 between a first extreme position 458 and a second extreme position 460, by rotation of said treaded rotatable axle 452 of said electromotor 451 in one rotational direction or in the opposite rotational direction.

Hereby the opening and the closing of the shutter mechanism 344 of the modular incubator chamber 300 can be controlled by making the rotatable axle 452 rotate in one of two rotational directions.

In alternative embodiments, the actuator 150 may be in the form of an electric solenoid or a piezo element.

The control of the shutter actuator 150 via the electro motor 451 may be performed with the control unit 650 as described below.

In an embodiment and in respect of one or more docking ports 402 of said docking station 400, said docking port 402 comprises one or more guide rails 462, wherein said second shutter mechanism engagement means 152 is being attached to said one or more guide rails 462 and is being configured to move along this/these guide rail(s) when being moved between its first extreme position 458 and its second extreme position 460.

The guide rails 462 aids in guiding the displacement movement of the second shutter mechanism engagement means 152.

In an embodiment of the modular incubator system 500 according to the first aspect of the present invention and in respect of one or more of said one or more docking ports 402 of said docking station 400, said shutter actuator 150 is being configured in such a way, that once a modular incubator chamber 300 is being docked in said docking port 402, then when said second shutter mechanism engagement means 152 is moving in a direction from its first extreme position 458 to its second extreme position 460, the magnetic force between said first magnet 368 of said first shutter mechanism engagement means 346 and said second magnet 450 of said second shutter mechanism engagement means 152 is configured for bringing said shutter mechanism 344 into its open configuration; and when said second shutter mechanism engagement means 152 is moving in a direction from its second extreme position 460 to its first extreme position 458, then the magnetic force between said first magnet 368 of said first shutter mechanism engagement means 346 and said second magnet 450 of said second shutter mechanism engagement means 152 is configured for bringing said shutter mechanism into its closed configuration.

In an embodiment and in respect of one or more docking ports 402 of said docking station 400, at least part of said one or more guide rails 462 is/are having an essentially horizontal extension.

In an embodiment and in respect one or more docking ports 402 of said docking station 400, said one or more guide rails 462, at a portion thereof corresponding to said first extreme position 458 of said second shutter mechanism engagement means 152, is inclined in a downward direction.

Hereby the magnetic force between the magnet 368 of the first shutter mechanism engagement means 346 and the second magnet 450 of the second shutter mechanism engagement means 152 will be gradually weakened when the second shutter mechanism engagement means 152 is approaching the first extreme position 458. This will reduce any the magnitude of uncontrolled movement, caused by magnetic attraction between the two magnets, when manually removing the modular incubator chamber 300 from its docking port 402 and in a situation in which the shutter mechanism is being in its closed configuration.

In an embodiment of the modular incubator system 500 according to the first aspect of the present invention and in respect of one or more of said one or more modular incubator chambers 300, said transparent window 316 of said housing 302 of said modular incubator chamber is having an elongate shape, such as an elongate and linear extension extending in a direction Y, transversal to said longitudinal direction X of said housing of said modular incubation chamber 300.

In an embodiment of the modular incubator system 500 according to the first aspect of the present invention and in respect of one or more of said one or more modular incubator chambers 300, said shutter mechanism 344 in its open configuration defines an elongate opening 370 defines an elongate opening 370 in said shutter mechanism 344, such as in said plate shaped shutter element 360, such as an elongate and linearly extending opening, for providing light into said interior 306 of said housing 302 of said modular incubator chamber; and wherein said elongate shape of said transparent window 316 of said housing 302 is being aligned with said elongate and linear extending opening 370 provided by said shutter mechanism 344, when said shutter mechanism in its open configuration.

Hereby the image capturing device 408 will be able to capture images of a multitude of viable biological materials which are being arranged in-line in a culture dish 310 in the interior 306 of the modular incubator chamber 300.

In an embodiment of the modular incubator system 500 according to the first aspect of the present invention and in respect of one or more of said one or more modular incubator chambers 300 and in respect of one or more of said one or more docking ports 402 of said docking station 400, said modular incubator chamber 300 is being configured to be docked in said docking port 402 with its first end 340 facing said docking port 402.

In an embodiment of the modular incubator system 500 according to the first aspect of the present invention and in respect of one or more of said one or more modular incubator chambers 300, said shutter mechanism 344 comprises a spring 345, wherein said spring is configured to maintain said shutter mechanism 344 in its closed configuration when not being acted upon by said external force.

Hereby the spring 345 will bring the shutter mechanism 344 into its closed configuration per default.

In an embodiment of the modular incubator system 500 according to the first aspect of the present invention and in respect of one or more of said one or more modular incubator chambers 300, said housing 302 and said lid 304 of said housing are being made of a material which is non-transparent to visible light.

In an embodiment of the modular incubator system 500 according to the first aspect of the present invention and in respect of one or more of said one or more modular incubator chambers 300, said lid 304 of said housing 302 is configured in such a way that once being in its closed configuration, said lid is blocking light from entering the interior of said housing, through the contact surface between said lid 304 and said housing 302.

These embodiments further minimize the influx of electromagnetic radiation or light into the interior 306 of the modular incubator chamber 300.

In an embodiment of the modular incubator system 500 according to the first aspect of the present invention and in respect of one or more of said one or more modular incubator chambers 300, said modular incubator chamber 300, in the interior 306 thereof, comprises a light source 372 for directing light to the area of the culture dish support 308 of said modular incubator chamber 300, thereby enabling illumination of a viable biological material in a situation of capturing images of said viable biological material.

The light source will improve the quality of images captured by the image capturing device 408 in an image capturing situation.

In an embodiment said light source 372 is being attached to said lid 304 of the housing 302 of said modular incubator chamber 300, at an inner side thereof.

Hereby light can easily be directed to a viable biological material which is arranged at a lower part of the interior 306 of the modular incubator chamber 300.

In an embodiment said light source 372 is being selected from the group of one or more LEDs, one or more laser diodes, one or more incandescent light bulbs.

It is to be understood, that as the invention seeks to minimize excessive exposure of electromagnetic radiation in the form of light to the viable biological material during incubation, the light source 372 should only be switched on in those short periods of time in which the image capturing unit 408 is in fact in the process of capturing an image.

In other embodiments light for illumination of the viable biological material being incubated in the interior of the modular incubator chamber 300 may be provided from the outside of the chamber 300 and may transmit light through a transparent window in the chamber 300. In such an embodiment, an addition shutter mechanism may be provided for shielding off light from being transmitted through such transparent window. Such shutter mechanism may have features similar to the shutter mechanisms discloses in respect of the first aspect of the present invention.

In an embodiment of the modular incubator system 500 according to the first aspect of the present invention and in respect of one or more of said one or more modular incubator chambers 300, said culture dish support 308 is defining a planar support surface for supporting said culture dish 310.

In an embodiment of the modular incubator system 500 according to the first aspect of the present invention and in respect of one or more of said one or more modular incubator chambers (300), said housing (302) of said modular incubator chamber (300), such as at an outer portion thereof, is being provided with electric connectors (322) for providing electric power and/or electric signals to said modular incubator chamber; and wherein in respect of one or more docking ports (402) of said docking station (400), said docking port is being provided with electric connectors (410), thereby allowing providing electric power and/or electric signals between said docking port (402) of said docking station (400) and a modular incubator chamber (300) being docked therein.

Hereby conveying of electric power or electric signals between said docking port 402 and said modular incubator chamber 300 is enabled.

In an embodiment of the modular incubator system 500 according to the first aspect of the present invention and in respect of one or more of said one or more modular incubator chambers 300, said lid 304 is being a hinged lid which is being connected to said housing of said modular incubator chamber via a hinge.

In an embodiment of the modular incubator system 500 according to the first aspect of the present invention and in respect of one or more of said one or more modular incubator chambers 300, said housing 302 of said modular incubator chamber 300 comprises a display 324 which is being configured to display information relating to an operational status of the incubation taking place in said modular incubator chamber.

In an embodiment of the modular incubator system 500 according to the first aspect of the present invention said image capturing device (408) comprises microscopic optics so as to enable capturing of microscope images.

Hereby magnified images may be captured which improves study of the morphological nature of the biological materials being incubated.

In an embodiment of the modular incubator system 500 according to the first aspect of the present invention the number of modular incubator chambers 300 of said modular incubator system 500 is selected from the ranges 1 - 100, such as 2 - 95, for example 5 - 90, e.g. 10 - 85, such as 15 - 80, for example 20 - 75, e.g. 25 - 70, 30 - 65, such as 35 - 60, e.g. 40 - 55 or 45 - 50.

In an embodiment of the modular incubator system 500 according to the first aspect of the present invention the number of docking ports 402 in said docking station 400 of said modular incubator system 500 is selected from the ranges 1 - 100, such as 2 - 95, for example 5 - 90, e.g. 10 - 85, such as 15 - 80, for example 20 - 75, e.g. 25 - 70, 30 - 65, such as 35 - 60, e.g. 40 - 55 or 45 - 50.

In an embodiment of the modular incubator system 500 according to the first aspect of the present invention the docking station 400 comprises said docking ports 402 in an arrangement of one or more shelves of adjacently positioned docking ports 402, wherein in case said docking station comprises two or more shelves, said shelves are being arranged above each other.

In an embodiment of the modular incubator system 500 according to the first aspect of the present invention in respect of one or more of said one or more modular incubator chambers 300, said modular incubator chamber comprises an incubation chamber engagement means 326 and wherein in respect of one or more docking ports 402 of said docking station 400, said docking port comprises a docking port engagement means 414, wherein said incubation chamber engagement means 326 is being configured to enter into engagement with said docking port engagement means 414 so as to provide ease and proper positioning and optionally also fixing said modular incubator chamber 300 in said docking port 402, as well as detaching said modular incubator chamber 300 from said docking port 402 of said docking station 400.

Hereby, easy and proper positioning and optionally also fixing said modular incubator chamber 300 in said docking port 402, as well as detaching said modular incubator chamber 300 from said docking port 402 of said docking station 400 is provided.

In an embodiment of the modular incubator system 500 according to the first aspect of the present invention the modular incubator system 500 comprises an image processing unit 660 for image processing of images captured by said image capturing device 408, wherein said modular incubator system 500 optionally furthermore comprises a data storage 658 for storing images captured by said image capturing units 408 and/or for storing images processed by said image processing unit 660.

An image processing unit is beneficial for manipulating the images captured, such as for adjusting contrast, for filtering and for generating time-lapse series of images.

In an embodiment one or more of said image capturing devices 408 of said docking ports 402 of said docking station is/are being coupled to an image processing unit 660.

In an embodiment of the modular incubator system 500 according to the first aspect of the present invention and in respect of one or more specific docking ports 402 of said docking station 400, said specific docking port comprises its own dedicated image capturing device 408 which is configured to only capture images relating to a modular incubator chamber 300 which is being docked in said specific docking port 402.

In an embodiment of the modular incubator system 500 according to the first aspect of the present invention and respect of a number N of adjacently arranged docking ports 402 of said docking station 400, said adjacently arranged docking ports share a common image capturing device 408 in the sense that one and only one image capturing device is responsible for capturing images relating to a modular incubator chamber 300 which is being docked in one of said N adjacently arranged docking ports 402, wherein said docking station comprises a displacement device 482 for enabling displacement of said common image capturing device 408 in relation to said N adjacently arranged docking ports 402 of said docking station 400.

Hereby one image capturing device is responsible for the capturing of images of biological materials being accommodated in different modular incubator chambers which are being docked in different docking port 402 of the docking station 400.

In an embodiment the number N is being an integer selected in the ranges of 2 - 25 or more, such as 4 - 22, for example 6 - 20, such as 8 - 18, such as 10 - 16 or 12 - 14.

Independently, one or more image capturing devices 408, preferably all image capturing devices 408 of the docking station 400 may comprise or be coupled to a displacement device 482, such as an electrically driven and remotely controlled displacement device 482 for enabling displacement of said common image capturing device 408 in a direction transversal to the longitudinal direction X of a modular incubator chamber 300 being docked in a docking port 402 with the view to enable such capturing device 408 to focus on more than one culture well in a culture dish 310 being accommodated in the interior of the modular incubator chamber 300, wherein such culture wells are arranged in such direction transversal to the longitudinal direction X.

In an embodiment of the modular incubator system 500 according to the first aspect of the present invention and in respect of one or more of said modular incubator chambers 300, said modular incubator chamber comprises in its interior 306 an electric heating element 318 for heating the interior of said modular incubator chamber, and wherein said modular incubator chamber comprises a power source 320 for providing power to said heating element 318, wherein said electric heating element 318 is being electrically connected to said power source 320.

In an embodiment said power source 320 is being an electric power source, such as a battery, for example a rechargeable battery.

In an embodiment said heating element 318 is being thermally connected to a heat distribution element for distributing heat dissipated in said heating element; wherein said heat distribution element is being arranged, at least partly, in the interior 306 of said modular incubator chamber 300.

In an embodiment the chamber comprises a thermostat 374 and an electric thermostatic circuit 376, wherein said electric heating element 318, said power source 320 and said thermostat 374 are being electrically connected in said electric thermostatic circuit 376 so as to enable thermostatic control of the temperature inside said modular incubator chamber 300.

The above embodiments provide for upholding a desirable and predetermined and optionally also optimum temperature in the interior 306 of the modular incubator chamber 300 in a situation where the modular incubator chamber is removed from its associated docking port 402 with the view to perform visual inspection and manual replenishing, removal or exchange of growth medium to the biological materials being incubated.

In an embodiment of the modular incubator system 500 according to the first aspect of the present invention and in respect of one or more of said one or more modular incubator chambers 300, said modular incubator chamber 300 comprises a chamber inlet opening for gas 312, wherein said chamber inlet opening for gas 312 is being in fluid connection with the interior 306 of said modular incubator chamber; and wherein said modular incubator chamber 300 furthermore comprises a chamber outlet opening for gas 314, wherein said chamber outlet opening for gas 314 is being in fluid connection with the interior 306 of said modular incubator chamber; and wherein in respect of one or more docking ports 402 of said docking station 400, said docking port 402 comprises a docking port outlet opening for gas 404 and a docking port inlet opening for gas 406; thereby enabling transfer of gas from said docking port 402 of said docking station 400 to the interior 306 of said modular incubator chamber 300 via said docking port outlet opening for gas 404 and said chamber inlet opening for gas 312; and thereby enabling transfer of gas from the interior 306 of said modular incubator chamber 300 to said docking port 402 of said docking station 400 via said chamber outlet opening for gas 314 and said docking port inlet opening for gas 406.

In an embodiment of the modular incubator system 500 according to the first aspect of the present invention and in respect of one or more of said one or more modular incubator chambers 300, and in respect of one or more of said one or more docking ports 402 of said docking station 400, the position of said chamber inlet opening for gas 312 of said housing 302 of said modular incubator chamber 300 and the position of said docking port outlet opening for gas 404 of said docking port 402 are adapted to each other in such a way that once docking said modular incubator chamber 300 in said docking port 402, said chamber inlet opening for gas 312 of said housing 302 of said modular incubator chamber 300 and said docking port outlet opening for gas 404 of said docking port 402 will be in fluid connection, thereby enabling transfer of gas from said docking port 402 to said modular incubator chamber 300; and wherein the position of said chamber outlet opening for gas 314 of said housing 302 of said modular incubator chamber 300 and the position of said docking port inlet opening for gas 406 of said docking port 402 are adapted to each other in such a way that once docking said modular incubator chamber 300 in said docking port 402, said chamber outlet opening for gas 314 of said housing 302 of said modular incubator chamber 300 and said docking port inlet opening for gas 406 of said docking port 402 will be in fluid connection, thereby enabling transfer of gas from said modular incubator chamber 300 to said docking port 402.

These embodiment ensure that gas having a desired composition can be delivered from a gas source 202 via a gas distribution system 204 to the interior 306 of the modular incubator chamber 300 via the docking port outlet opening for gas 404 and the chamber inlet opening for gas 312, and gas from the interior 306 of the modular incubator chamber 300 can be returned to the gas source 202 via the chamber outlet opening for gas 314 and the docking port inlet opening for gas 406.

In an embodiment of the modular incubator system 500 according to the first aspect of the present invention the docking port outlet opening for gas 404 of said docking port 402 comprises a valve 4 and said chamber inlet opening for gas 312 of said housing 302 comprises a valve 2; and said chamber outlet opening for gas 314 comprises a valve 2 and said docking port inlet opening for gas 406 of said docking port 402 comprises a valve 4.

Hereby can be assured that gas will only flow into the docking port 402 once a modular incubator chamber 300 is being arranged in that docking port 402. In other words, no gas will flow into the docking port 402 unless a modular incubator chamber 300 is being docked therein. Moreover, this embodiment assures that once a modular incubator chamber 300 is being removed form a docking port, no atmospheric air will enter through the chamber inlet opening for gas 312 and the chamber outlet opening for gas 314 of that chamber 300.

Accordingly, there will be no contamination of the gas atmosphere being present in the interior 306 of the modular incubator chamber 300, once that chamber 300 is being removed from its docking port 402.

In an embodiment of the modular incubator system 500 according to the first aspect of the present invention and in respect of one or more of said one or more modular incubator chambers 300, said valve 2 of said chamber inlet opening for gas 312 and said valve 2 of said chamber outlet opening for gas 314 each comprises a valve body 6 having a front end 10, a rear end 12 and a through-going channel 14 therein, and a spring-loaded displaceable valve element 8, wherein said displaceable valve element 8 is being arranged in said through-going channel 14; wherein said displaceable valve element 8 is being configured to be displaceable in said through-going channel 14 of said valve body 6 in such a way, that when not acted upon by an external force, said spring-loaded displaceable valve element 8 is not being displaced in said through-going channel 14 of said valve body 6, thereby making said valve attain a closed configuration blocking passage of gas through said through-going channel 14, and in such a way, that when acted upon by an external force, said spring-loaded displaceable valve element 8 is being displaced in said through-going channel 14 of said valve body 6, thereby making said valve 2 attain an open configuration, allowing passage of gas through said through-going channel 14; and
wherein in in respect of one or more of said one or more docking ports 402 of said docking station 400, said valve 4 of said docking port outlet opening for gas 404 and said valve 4 of said docking port inlet opening for gas 406 each comprises a valve body 16 having a front end 20, a rear end 22 and a through-going channel 24 therein, and a spring-loaded displaceable valve element 18, wherein said displaceable valve element 18 is being arranged in said through-going channel 24; wherein said displaceable valve element 18 is being configured to be displaceable in said through-going channel 24 of said valve body 16 in such a way, that when not acted upon by an external force, said spring-loaded displaceable valve element 18 is not being displaced in said through-going channel 24 of said valve body 16, thereby making said valve attain a closed configuration, blocking passage of gas through said through-going channel 24, and in such a way, that when acted upon by an external force, said spring-loaded displaceable valve element 18 is being displaced in said through-going channel 24 of said valve body 16, thereby making said valve 4 attain an open configuration, allowing passage of gas through said through-going channel 24.

Hereby, each of the two valves 2,4 will be able to change configuration between an open and a closed configuration by displacement of the respective valve element 8,18 in the associated valve body 6,16.

In an embodiment of the modular incubator system 500 according to the first aspect of the present invention and in respect of one or more of said one or more docking ports 402 of said docking station 400, and in respect of one or more of said one or more modular incubator chambers 300 said valves 2,4 are having dimensions and geometries in such a way that once docking said modular incubator chamber 300 in said docking port 402 of said docking station 400, said displaceable valve element 8 of said valve 2 and said displaceable valve element 18 of said valve 4 will displace each other into their respective valve bodies 6,16, thereby opening said valves 2,4 of said docking port outlet opening for gas 404 and said chamber inlet opening for gas 312; and thereby opening said valves 2,4 of said chamber outlet opening for gas 314 and said docking port inlet opening for gas 406.

Hereby, each of the two valves 2,4 will open the other valve 4,2 once being brought into contact with each other by making their respective front ends 10,20 meet.

In an embodiment of the modular incubator system 500 according to the first aspect of the present invention and in respect of one or more of said docking ports 402 of said docking station 400 of said modular incubator system 500, preferably in respect of all said docking ports 402, said docking port outlet opening for gas 404 comprises a flow restrictor for restricting the magnitude of flow of gas flowing into said docking port 402.

In one embodiment the flow restrictor may comprises a tube through which the gas is conveyed to said docking port 402, wherein said tube optionally is having a cross-sectional area selected from the ranges of 0.2 - 8 mm², such as 0.5 - 7 mm², for example 1 - 6 mm², such as 2 - 5 mm² or 3 - 4 mm²; and/or the length of said tube is optionally selected from the ranges of 5 - 30 mm, such as 8 -25 mm, for example 10 - 22 mm, e.g. 15 - 20 mm.

Such a flow restrictor aids in balancing the flow of gas through the docking ports 402 comprising a modular incubator chamber 300 with the capacity of the gas supply system 200 and thereby also aids in making the flow of gas through the different docking ports 402 equal to each other.

In an embodiment of the modular incubator system 500 according to the first aspect of the present invention the docking station 400 comprises a gas distribution system 204 for supplying gas to and from one or more of said one or more docking ports 402, wherein said gas distribution system 204 comprises a main gas supply line 210 and a main gas return line 212, wherein in respect of one or more of said docking ports 402, said docking port inlet opening for gas 404 is being fluidly connected to said main gas supply line 210, and said docking port outlet opening for gas 406 is being fluidly connected to said main gas return line 212.

In one embodiment said gas distribution system 204 comprises a number of manifold pairs 214, wherein each manifold pair comprises an inlet manifold 216 and an outlet manifold 218, wherein said inlet manifold 216 is being fluidly connected to said main gas supply line 210 and wherein said outlet manifold 218 is being fluidly connected to said main gas return line 212; wherein each manifold pair 214 is connected to one or more docking ports 402 of said docking station 400 in such a way that in respect of a specific manifold pair 214, and in respect of said one or more docking ports 402 being connected thereto, said docking port outlet opening for gas 404 of said docking port 402 is being fluidly connected to said inlet manifold 216, and said docking port inlet opening for gas 406 of said docking port 402 is being fluidly connected to said outlet manifold 218.

In one embodiment said docking station 400 comprises a gas supply system 200, wherein said gas supply system 200 comprises a gas source 202 and said gas distribution system 204, wherein said gas source comprises a supply gas outlet 206 and a return gas inlet 208, wherein said supply gas outlet 206 of said gas source 202 is being fluidly connected to said main gas supply line 210 of said gas distribution system 204, and wherein said return gas inlet 208 of said gas source 202 is being fluidly connected to said main gas return line 212 of said gas distribution system 204.

In these embodiments comprising a gas distribution system 204 it is possible to supply gas from a gas source 202 to the docking ports 402 via the main gas supply line 210 and to return gas from the docking ports to the gas source 202 via the main gas return line 212.

In an embodiment of the modular incubator system 500 according to the first aspect of the present invention, the gas source 202 of said gas supply system 200 comprises a gas mixing box 242 fluidly connected to said supply gas outlet 206 and said return gas inlet 208 of said gas source, wherein said main gas supply line 210 of said gas distribution system 204 is being fluidly connected to said supply gas outlet 206, and wherein said main gas return line 212 of said gas distribution system 204 is being fluidly connected to said return gas inlet 208 of said gas source 202, thereby forming a flow loop 244 comprising said gas distribution system 204 and said gas mixing box 242; wherein said flow loop comprises a pump 246.

Hereby circulating gas in said loop, and also through the gas distribution system 204 of the docking station 400 is possible.

The purpose of the gas source is to provide and deliver a desired gas composition to the gas distribution system 204 including the various docking ports 402 of the docking station 400 with the view to supply this gas to the interior 306 of the modular incubator chambers 300.

In one embodiment of this embodiment the pump 246 is being arranged downstream in relation to said main gas return line 212.

In one embodiment the flow loop 244 comprises a pump oscillation damper 247, wherein said pump oscillation damper optionally is being arranged immediately downstream in relation to said pump 246.

The pump oscillation damper will equalize small and rapid pressure variations caused by each pump stroke of the pump.

In an embodiment of the modular incubator system 500 according to the first aspect of the present invention, the flow loop 244 comprises a pressure sensor, such as a differential pressure sensor 248 for sensing the pressure of gas supplied to said main gas supply line 210 of said gas distribution system 204, wherein said pressure senor 248 optionally is being arranged immediately upstream in relation to said main gas supply line 210 of said gas distribution system 204.

The pressure sensor 248 allows for regulating the pump 246 in order to maintain a desired pressure in the flow loop 244 by feedback.

In one embodiment the pressure sensor 248 is being a differential pressure sensor, sensing a pressure relative to the pressure of the return gas inlet 208.

In one embodiment the flow loop 244 comprises a release valve 249 for enabling pressure relief in said flow loop, wherein said release valve optionally is being arranged immediately downstream in relation to said main gas return line 212 of said gas distribution system 402.

The pressure release valve 249 enables improved control of the pressure in the flow loop 344.

In an embodiment of the modular incubator system 500 according to the first aspect of the present invention, the gas mixing box 242 comprises an inlet for N₂ gas 250; and an inlet for CO₂ gas 251, wherein said inlet for N₂ gas 250 is fluidly connected to an N₂ valve 252 for regulating the inflow of N₂, and an N₂ mass flow sensor 253 arranged downstream of said N₂ valve 252 for sensing the amount of N₂ flowing into said gas mixing box 242; and wherein said inlet for CO₂ gas 251 is fluidly connected to a CO₂ valve 254 for regulating the inflow of CO₂, and an CO₂ mass flow sensor 255 arranged downstream of said CO₂ valve 254 for sensing the amount of CO₂ flowing into said gas mixing box 242.

Herby it is possible to control the inlet of N₂ gas and the inlet of CO₂ gas into the gas mixing box 242 with the view to obtain a desired, predetermined and optimum gas composition in the gas mixing box 242.

In an embodiment of the modular incubator system 500 according to the first aspect of the present invention, the flow loop 244 comprises a mass flow sensor 256 arranged at an upstream position in relation to said gas mixing box 242 for sensing the amount of return gas entering said gas mixing box.

Information relating to the amount of return gas entering said gas mixing box is used for determining the total amount of N₂ gas and CO₂ gas which needs to be introduced into the gas mixing box 242.

In an embodiment of the modular incubator system 500 according to the first aspect of the present invention, the gas source 202 comprises an O₂ sensor 258 for sensing the concentration of O₂ exiting said gas distribution system 204; and wherein said gas source 202 comprises a CO₂ sensor 260 for sensing the concentration of CO₂ exiting said gas distribution system 204, wherein said O₂ sensor and/or said CO₂ sensor optionally is/are being arranged downstream in relation to said pump 246.

Information relating to the concentration of O₂ and the concentration of CO₂ exiting said gas distribution system 204 is used for determining the specific amount of N₂ gas and the specific amount of CO₂ gas which needs to be introduced into the gas mixing box 242.

In an embodiment of the modular incubator system 500 according to the first aspect of the present invention, the gas source 202 comprises a temperature sensor 262 for sensing the temperature of gas circulating in said flow loop 244, wherein said temperature sensor optionally is being arranged downstream in relation to said pump 246, preferably at a position corresponding to the position of said O₂ sensor 258.

In an embodiment of the modular incubator system 500 according to the first aspect of the present invention, the gas source 202 comprises a pressure sensor 264 for sensing the absolute pressure in said flow loop 244 wherein said pressure sensor optionally is being arranged downstream in relation to said pump 246, preferably at a position corresponding to the position of said CO₂ sensor 260.

The temperature sensor 262 and pressure sensor 264 are useful for performing compensation of the readings of the O₂ sensor 258 due to temperature sensitivity thereof and the readings of the CO₂sensor 260 due to sensitivity thereof towards pressure.

In an embodiment of the modular incubator system according to the first aspect of the present invention, the flow loop 244 comprises a UV sanitizer 266 for sanitizing gas flowing in said flow loop 244 via electromagnetic radiation in the UV range, wherein said UV sanitizer optionally being arranged immediately downstream in relation to said main gas return line 212.

In an embodiment of the modular incubator system 500 according to the first aspect of the present invention, the gas source 202 comprises one or more filters 268, such as HEPA and/or VOCs filters, wherein such a filter is being arranged immediately upstream in relation to said main gas supply line 210, and/or wherein such a filter is being arranged immediately upstream in relation to the inlet for N₂ gas 250 into said gas mixing box 242; and/or wherein such a filter is being arranged immediately upstream in relation to the inlet for CO₂ gas 251 into said gas mixing box 242.

In an embodiment of the modular incubator system 500 according to the first aspect of the present invention, the gas source 202 comprises a gas mixing control system 270, wherein said gas mixing control system is electrically connected to one or more of the following sensors for receiving sensing signals therefrom: said N₂ mass flow sensor 253 for sensing the amount of N₂ flowing into said gas mixing box; said CO₂ mass flow sensor 255 for sensing the amount of CO₂ flowing into said gas mixing box; said mass flow sensor 256 for sensing the amount of return gas entering said gas mixing box; said O₂ sensor 258 for sensing the concentration of O₂ exiting said main gas return line 212 of said gas distribution system 204; said CO₂ sensor 260 for sensing the concentration of CO₂ exiting said main gas return line 212 of said gas distribution system 204; said temperature sensor 262 for sensing the temperature circulating in said flow loop 244; said pressure sensor 264 for sensing an absolute pressure in said flow loop 244, said pressure sensor 248 for sensing the pressure of gas supplied to said gas main gas supply line 210 of said distribution system 204.

This embodiment enables gaining information of various parameters which are to be used in providing a feed back when controlling the operation of the gas source 202.

In an embodiment of the modular incubator system 500 according to the first aspect of the present invention, the gas mixing control system 270 is electrically connected to one or more of the following elements for control thereof: said N₂ valve 252 for regulating the inflow of N₂ into said gas mixing box 242; said CO₂ valve 254 for regulating the inflow of CO₂ to said gas mixing box 242; said pump 246 for circulating gas in said flow loop 244; said release valve 249.

This embodiment enables providing a feed back when controlling the operation of the gas source 202.

In one embodiment the gas mixing control system 270 is being configured to receive input from said pressure sensor 248 and on the basis thereof control said pump 246, optionally also to activate said release valve 249 in order to maintain a desired and predetermined pressure of gas supplied to said main gas supply line 210 of said gas distribution system 204.

Hereby the pressure in the flow loop 244 can be controlled.

In an embodiment of the modular incubator system 500 according to the first aspect of the present invention, the gas mixing control system 270 is being configured to receive input from said mass flow sensor 256, and on the basis on said input to determine the total amount of CO₂ gas and N₂ gas needed to be supplied via said inlet for CO₂ gas 251 and via said inlet for N₂ gas 250 according to desired and predetermined criteria.

In an embodiment of the modular incubator system 500 according to the first aspect of the present invention, the gas mixing control system 270 is being configured to receive input from said CO₂ sensor 260 and said O₂ sensor 258, and on the basis of the CO₂ concentration sensed, is configured to control said CO₂ valve 254, by transmitting a control signal thereto, and thereby regulating the inflow of CO₂ gas in order to reach a desired and predetermined CO₂ concentration, and wherein subsequently, said gas mixing control system 270 on the basis of the O₂ concentration sensed, is configured to control said N₂ valve 252, by transmitting a control signal thereto, and thereby regulating the inflow of N₂ gas in order to reach a desired and predetermined O₂ concentration.

In an embodiment of the modular incubator system 500 according to the first aspect of the present invention, the gas mixing control system 270 is configured to use the input from said temperature sensor 262 for compensating the temperature sensitivity of said O₂ sensor 258.

In an embodiment of the modular incubator system 500 according to the first aspect of the present invention, the gas mixing control system 270 is configured to use the input from said pressure sensor 264 for compensating the pressure sensitivity of said CO₂ sensor 260.

In an embodiment of the modular incubator system 500 according to the first aspect of the present invention, the gas mixing control system 270 is being configured to maintain a pressure of gas supplied to said main gas supply line 210 of said gas distribution system 204, relative to the ambient atmospheric pressure, of 3 - 20 mbar, such as 5 - 18 mbar, such as 10 - 15 mbar above that ambient atmospheric pressure.

In an embodiment of the modular incubator system 500 according to the first aspect of the present invention, the gas mixing control system 270 is being configured to maintain a CO₂ concentration of gas entering said main gas supply line 210 of said gas distribution system 204 in the range of 5 - 10%, such as 6 - 9 % or 7 - 8 %; and/or an O₂ concentration of gas entering said main gas supply line 210 of said gas distribution system 204 in the range of 5 - 10%, such as 6 - 9 % or 7 - 8 %.

In an embodiment of the modular incubator system 500 according to the first aspect of the present invention said modular incubator system 500 comprises a control unit 650 for controlling the operation of said modular incubator system 500.

In an embodiment said control unit 650 is being coupled to an input device 652, such as an alphanumerical input device for allowing a user to provide settings input relating to a desired operational protocol of said modular incubator system.

In an embodiment said control unit 650 is being coupled to a display unit 654 for displaying, to a user, information relating to settings and/or operational status of said modular incubator system 300.

In an embodiment of the modular incubator system 500 according to the first aspect of the present invention and in respect of one or more docking ports 402 of said docking station 400 said control unit 650 is being configured for independently controlling one or more of the following: the temperature in the interior 306 of said modular incubator chamber 300 by controlling said electric heating element 318, said thermostat (374 or said thermostatic circuit 376, provision of power to said electric power source 320; provision of signals to said display 324 of a modular incubator chamber 300 being docked in said docking port 402, switching on and off of said active light source 352 of a modular incubator chamber 300 being docked in said docking port 402, or adjusting light intensity emitted therefrom, said image capturing device 408 of a docking port 402, said displacement device 482 for displacing said image capturing device 408, said shutter actuator 150, optionally via said electromotor 451, said gas mixing control system 270; and said image processing unit 660.

When controlling a modular incubator chamber 300 this way, electric signals or electric power is accordingly being provides from the control unit 650 via the electric connectors 410 of the docking port 402 wherein the modular incubator chamber 300 is being docked and also via the electric connectors 322 of the modular incubator chamber 300.

In an embodiment said control unit 650 is being coupled to a data processing unit 656 and optionally also to a data storage 658 for aiding in handling information during controlling of said modular incubator system.

In an embodiment said control unit 650 is being configured for conducting automatic operation of said modular incubator system 500 by configuring said control unit 650 to independently control one or more of the following: the temperature in the interior 306 of said modular incubator chamber 300 by controlling said electric heating element 318, said thermostat (374 or said thermostatic circuit 376, provision of power to said electric power source 320; provision of signals to said display 324 of a modular incubator chamber 300 being docked in said docking port 402, switching on and off of said active light source 352 of a modular incubator chamber 300 being docked in said docking port 402, or adjusting light intensity emitted therefrom, said image capturing device 408 of a docking port 402, said displacement device 482 for displacing said image capturing device 408, said shutter actuator 150, optionally via said electromotor 451, said gas mixing control system 270; and said image processing unit 660.

The automatic operation is being conducted according to predetermined control criteria and instructions.

In an embodiment control unit 650 is being configured for enabling time lapse capturing of images by said image capturing devices 408.

In the above embodiments the operation of the modular docking system 500 can easily be controlled centrally.

### The second aspect of the present invention

In a second aspect the present invention relates to a modular incubator chamber 300 comprising:
a housing 302 having a first end 340 and a second end 342, thereby defining a longitudinal direction X between said first end and said second end;
wherein said housing comprises a lid 304, wherein said lid is being configured to be able to shift between an open configuration allowing access to the interior 306 of said modular incubator chamber 300 and a closed configuration, sealing off access to the interior of said modular incubator chamber;
wherein said modular incubator chamber 300, at said interior 306 thereof, comprises a culture dish support 308 for positioning a culture dish 310 with the view to accommodate one or more biological materials M within the housing 302 of said modular incubator chamber 300;
wherein said housing 302 of said modular incubator chamber 300 comprises a transparent window 316 for enabling capturing of images of a biological material M being accommodated in the interior thereof, through said transparent window;
wherein said housing 302 of said modular incubator chamber 300 comprises a shutter mechanism 344, wherein said shutter mechanism is configured to be able to shift between an open configuration and a closed configuration and vice versa;
wherein said shutter mechanism 344 comprises a first shutter mechanism engagement means 346 for enabling shifting said shutter mechanism between its open configuration and its closed configuration and vice versa, upon exerting a force thereto;
wherein said shutter mechanism is being arranged relative to said transparent window 316 of said housing 302 in such a way that in its open configuration, said shutter mechanism 344 is enabling transmittal of light, from the outside, into the interior 306 of said modular incubator chamber 300 through said transparent window 316; and in such a way that in its closed configuration, said shutter mechanism 344 is blocking transmittal of light, from the outside, into the interior 306 of said modular incubator chamber 300 through said transparent window 316.

In an embodiment of the modular incubator chamber 300 according to the second aspect of the present invention said incubator chamber 300 is comprising features as defined in respect of the modular incubator chamber (300) of the modular incubator system (500) according to the first aspect of the present invention.

### The third aspect of the present invention

In a third aspect the present invention relates to a docking station 400 for docking one or more modular incubator chambers 300; wherein said docking station comprises one or more docking ports 402 for receiving a housing 302 of one or more of said incubator chambers 300; wherein in respect of one or more docking ports 402 of said docking station, said docking port comprises an image capturing device 408 for capturing an image of the interior 306 of a modular incubator chamber 300, once being docked in said docking port 402; wherein one or more of said docking ports 402 comprises a shutter actuator 150, such as an electric shutter actuator which, upon being provided with a signal thereto, such as an electric signal, is being configured to exert a force to said first shutter mechanism engagement means 346 of an associated incubator chamber 300, in order to shift the configuration of said shutter mechanism 344 of said associated incubator chamber 300 between said open configuration and said closed configuration, and vice versa, when said incubator chamber 300 is being docked in said docking port 402.

In an embodiment of the docking station 400 according to the third aspect of the present invention said docking station 400 is comprising features as defined in respect of the docking station 400 of the modular incubator system (500) according to the first aspect of the present invention.

### The fourth aspect of the present invention

In a fourth aspect the present invention provides a use of a modular incubator system 500 according to the first aspect of the present invention for incubating a viable biological material.

In an embodiment of the use according to the fourth aspect of the present invention said biological material is being an oocyte or an embryo, such as a human oocyte or a human embryo.

### The fifth aspect of the present invention

In a fifth aspect the present invention provides a use of a modular incubator chamber 300 according to the second aspect of the present invention for incubating a viable biological material.

In an embodiment of the use according to the fifth aspect of the present invention said biological material is being an oocyte or an embryo, such as a human oocyte or a human embryo.

### The sixth aspect of the present invention

In a sixth aspect the present invention provides a use of a docking station 400 according to according to the second aspect of the present invention for incubating a viable biological material.

In an embodiment of the use according to the sixth aspect of the present invention said biological material is being an oocyte or an embryo, such as a human oocyte or a human embryo.

### The seventh aspect of the present invention

In a seventh aspect the present invention provides method of incubating a viable biological material, wherein said method comprises:
i) providing a modular incubator system 500 according to the first aspect of the present invention;
ii) providing a viable biological material;
iii) arranging said viable biological material in a culture dish 310 and subsequently arranging said culture dish in the interior 306 of a modular incubator chamber 300 of said modular incubator station 400;
iv) docking said modular incubator chamber 300 in a docking port 402 of said docking station 400 of said incubator system 500;
v) allowing said viable biological material to be incubated in said modular incubator chamber 300, while ensuring that said shutter mechanism 344 of said modular incubator chamber 300 is being in its closed configuration;
vi) whenever desired, shifting the configuration of said shutter mechanism 344 of said modular incubator chamber 300 to its open configuration and allowing said image capturing device 408 to capture one or more images of said biological material being accommodated in said culture dish 310;
vii) optionally, upon finishing capture of images of said biological material, shifting the configuration of said shutter mechanism 344 of said modular incubator chamber 300 to its closed configuration.

In an embodiment of the method according to the seventh aspect of the present invention said method further comprising the step of:
viii) removing said incubator chamber 300 from said docking port 402 of said docking station 400, when desired, in order to manually inspect the viable biological material, and optionally also to remove, add or exchange growth medium/media in said culture dish 310.

It is noted that in the appended claims relating to the second aspect of the present invention, viz. the modular incubator chamber, reference is made that features of this modular incubator chamber may be as defined in respect of the claims relating to the first aspect of the present invention, viz. the modular incubator system.

This shall be construed to mean that embodiment of the modular incubator chamber per se may be as defined in the claims relating to embodiments of the modular incubator system.

This shall also be construed to mean that to the extent that an interrelationship between the modular incubator chamber and the docking station or a docking port thereof is defined in such embodiments relating to the modular incubator system, the corresponding embodiment of the modular incubator chamber, claimed by reference to the modular incubator system, shall be considered suitable to enter into such interrelationship.

Likewise, it is noted that in the appended claims relating to the third aspect of the present invention, viz. the docking station, reference is made that features of this docking station may be as defined in respect of the claims relating to the first aspect of the present invention, viz. the modular incubator system.

This shall be construed to mean that embodiment of the docking station per se may be as defined in the claims relating to embodiments of the modular incubator system.

This shall also be construed to mean that to the extent that an interrelationship between the modular incubator chamber and the docking station or a docking port thereof is defined in such embodiments relating to the modular incubator system, the corresponding embodiment of the docking station, claimed by reference to the modular incubator system, shall be considered suitable to enter into such interrelationship.

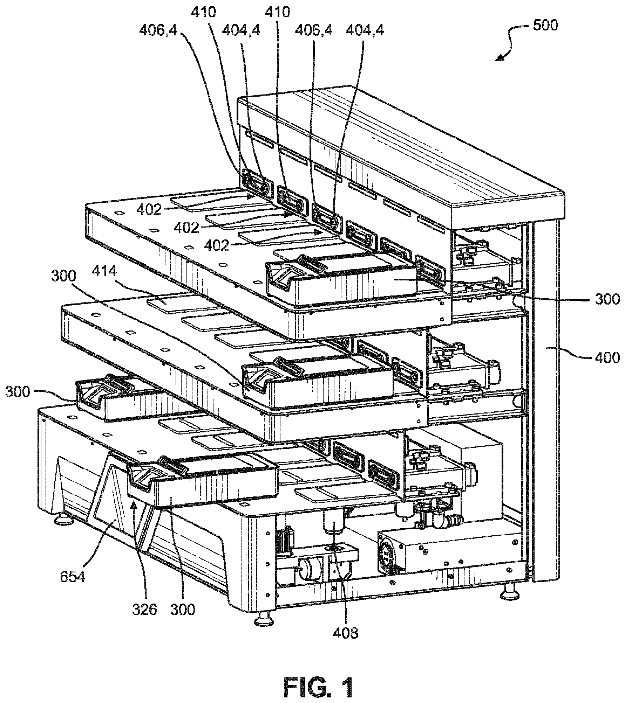

Referring now to the figures for better illustrating the present invention, Fig. 1 is a perspective view illustrating the general concept of designing an incubator as a modular incubator system comprising a plurality of modular incubator chambers in combination with a docking station having a plurality of docking ports.

Accordingly, Fig. 1 shows the modular incubator system 500 for incubating a viable biological material. The modular incubator system 500 comprising a docking station 400 in combination with a number of modular incubator chambers 300. The docking station 400 comprises a plurality of docking ports 402. Each docking port 402 is configured for receiving and holding a modular incubator chamber 300. Each docking port 402 comprises docking port engagement means 414 which is configured to enter into engagement with a corresponding incubation chamber engagement means 326 arranged underneath each modular incubation chamber 300.

In Fig. 1 it is seen that the docking station 400 of the incubator system 500 comprises three shelves arranged above each other and each comprising six docking ports 402. Three of these docking ports 402 have been occupied by a modular incubator chamber 300 and a fourth modular incubation chamber is on its way to be docked in a docking port 402.

The arrangement of an incubator for IVF procedures as an incubator system 500 comprising a plurality of modular incubator chambers 300 in combination with a docking station 400 allows for having, in a single apparatus, a relative huge number of incubations taking place under individual incubations environments, such as under an individual chemical environment in relation to e.g. gaseous atmosphere and composition of growth medium, and under an individual physical environment in relation to e.g. temperature.

Hereby it is possible to conduct a relatively large number of parallelly conducted incubations under similar conditions in the individual modular incubator chambers, while altering only one parameter from one modular incubator chamber to another. The difference in development of the viable biological material being incubated in the various modular incubator chambers can then be assigned to the one incubation parameter that is altered from one modular chamber to the other.

This allows for determining optimum growth conditions of an embryo or an oocyte being incubated.

Whenever it is needed to change or add growth medium to the biological being incubated or whenever other manual procedures are needed in respect of a specific modular incubator chamber, that chamber 300 is simple removed from its respective docking port 402 of the docking station 400 and transferred to a laboratory bench where such manual procedures can be conducted.

However, during most of the time, the modular incubator chamber 300 will be docked in a docking port 402 of the docking station 400.

Image capturing means 408 are provided in respect of one or more of the docking ports 402 of the docking station 400. The image capturing device(s) 408 of the docking station 400 provide(s) for monitoring the morphological changes taking place during incubation.

The image capturing device(s) 408 may be configured for automatically capturing of images of the biological material being incubated in a modular incubator chamber 300.

In order to enable image capturing of a biological material being accommodated in the interior of a modular incubator chamber by an image capturing device being arranged in the docking station 400 it is clear that the modular incubator chamber must allow transmittal of light through the housing of the modular incubator chamber.

Such transmittal of light through the housing of the modular incubator chamber is attained by providing the housing of the modular incubator chamber 300 with a transparent window which allows for an image capturing device, located outside the interior of the chamber 300, to capture images of a viable biological material being accommodated in the interior of that incubator chamber 300.

In recent years it has been generally acknowledged that embryos and oocytes are extremely sensitive in a detrimental way to all kinds of external impacts during incubation thereof.

Such detrimental impact may relate to physical impacts upon being physically moved. However, it has been suggested that also exposure to light may have a detrimental impact on the quality of the incubation of an oocyte or an embryo.

Accordingly, with the modular incubator system described above, it in inevitably, that at least in the operation of moving of the modular incubator chamber 300 from the docking port 402 of the docking station 400 to the work bench for conducting manual procedures, the biological material being incubated therein will be exposed to light entering through the transparent window in the housing of the modular incubator chamber.

The present invention seeks to solve this problem.

Accordingly, in the first aspect of the present invention an improved modular incubator system is provided.

The modular incubator system 500 according to the first aspect of the present invention for incubating a viable biological material comprises: one or more modular incubator chambers 300 in combination with a docking station 400.

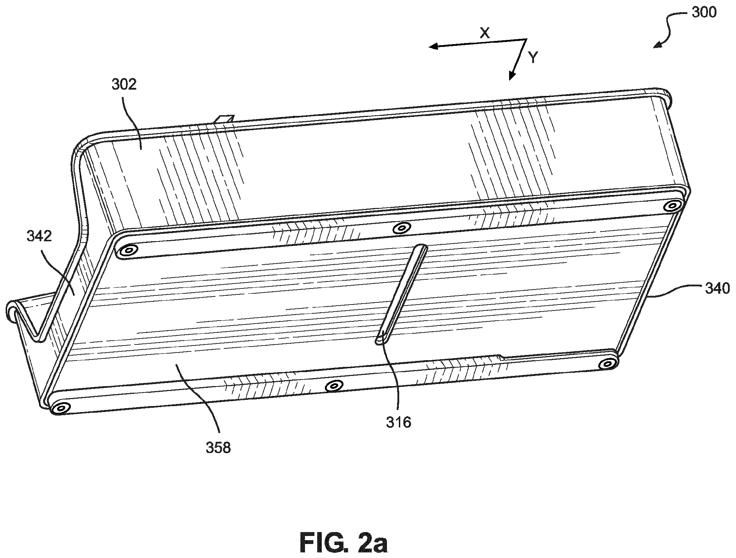

Details of the modular incubator chambers 300 of the docking system 500 according to the first aspect will now be described with reference to Fig. 2a - 3b, 5a, 5b and 6b.

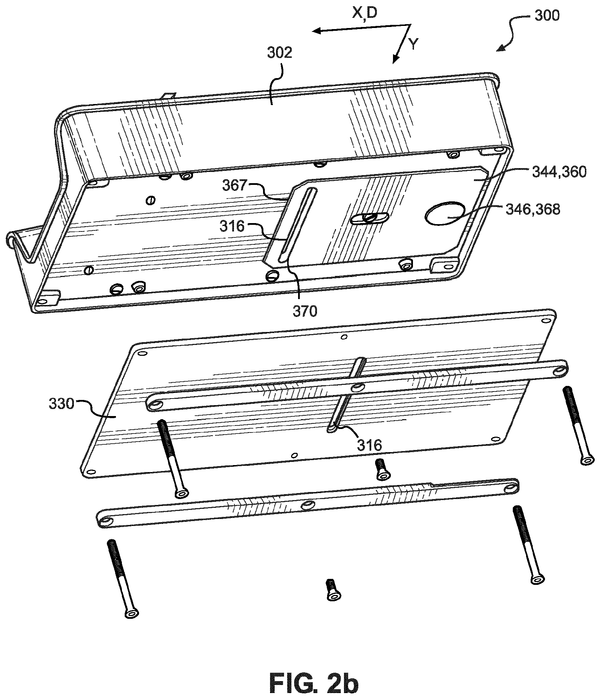

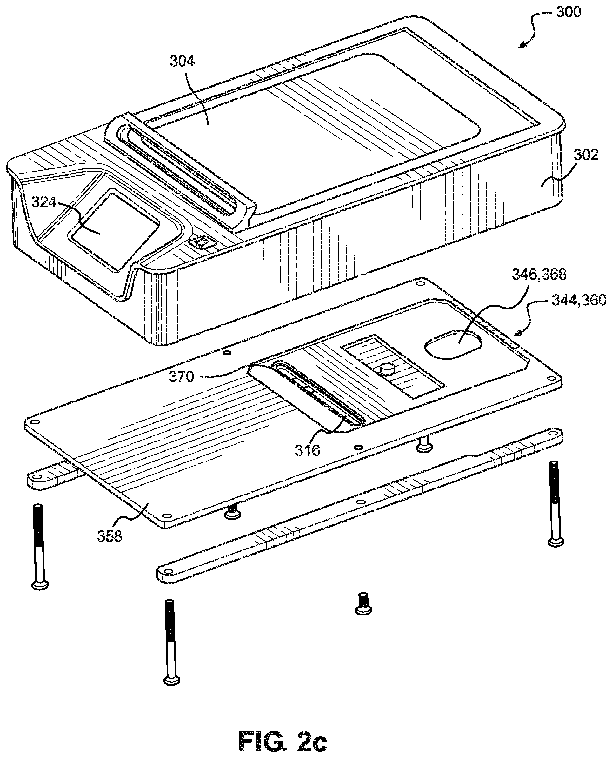

Fig. 2a is a bottom perspective view showing a modular incubator chamber of the modular incubator system of the invention. Fig. 2b is a partly exploded bottom perspective view showing the modular incubator chamber of Fig. 2a, and Fig. 2c is a partly exploded top perspective view showing the modular incubator chamber of Fig. 2a and Fig. 2b.

Fig. 2a illustrates the modular incubator chamber 300 comprising a housing 302. The housing 302 is having a first end 340 and a second end 342 which define a longitudinal direction X between the first end and the second end.

At a bottom part 358 of the housing 302 the housing comprises a transparent window 316.

The transparent window 316 enables capturing of images of a biological material which is being accommodated in the interior of the housing 302, through that transparent window.

A shutter mechanism 344 is being arranged relative to the transparent window 316 of said housing in such a way that in its open configuration, the shutter mechanism 344 is enabling transmittal of light, from the outside, into the interior 306 of the modular incubator chamber through the transparent window 316; and in such a way that in its closed configuration, the shutter mechanism 344 is blocking transmittal of light, from the outside, into the interior 306 of said modular incubator chamber through said transparent window 316.

Fig. 2b shows in a partly exploded perspective view that the housing 302 of the modular incubator chamber 300 comprises a shutter mechanism 344.

The shutter mechanism 344 is configured to be able to shift between an open configuration and a closed configuration and vice versa.

In the embodiment illustrated in Fig. 2b this is brought about by providing the shutter mechanism 344 with a plate shaped shutter plate 360 and by displacing the plate shaped shutter element 360 of the shutter mechanism 344 along the direction X, D as will be further described below.

The shift of the shutter mechanism 344 between its open configuration and its closed configuration is attained by exerting a force to the first shutter mechanism engagement means 346 as further described below.

Fig. 2c shows in a perspective view that the housing 302 of the modular incubator chamber 300 comprises a lid 304.

The lid is being configured to be able to shift between an open configuration allowing access to the interior of the modular incubator chamber 300 and a closed configuration, sealing off access to the interior of said modular incubator chamber.

The lid 304 is a hinged lid which is being connected to the housing 302 of the modular incubator chamber via a hinge.

Also seen in Fig. 2c is that the housing 302 of the modular incubator chamber 300 comprises a display 324 for displaying information relating to the incubation operation taken place in that incubator chamber 300.

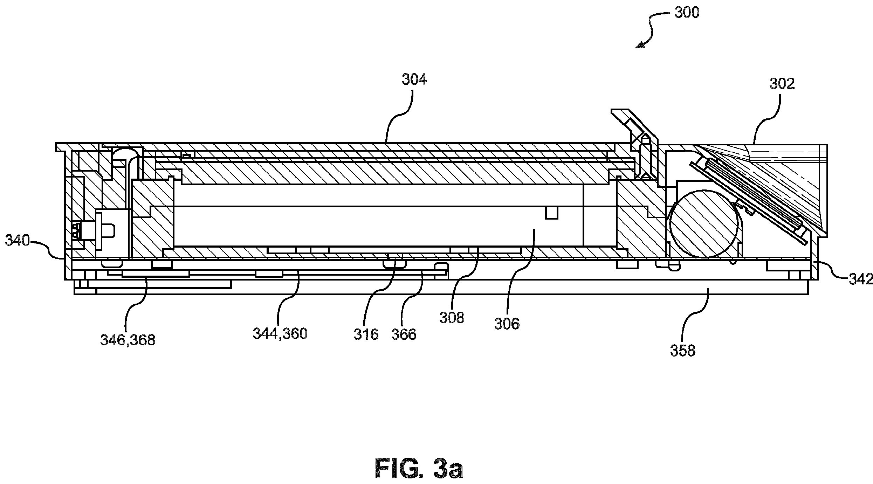

Fig. 3a is a cross-sectional view illustrating the the modular incubator chamber illustrated in Fig. 2a, 2b and 2c.

Fig. 3a shows the modular incubator chamber with the lid 304 and a culture dish support 308 arranged at the interior 306 of the housing 302 of the modular incubator chamber 300.

The dish support 308 allows for positioning a culture dish 310 in the interior 306 of the modular incubator chamber 300 with the view to accommodate one or more biological materials M within the housing 302 of that modular incubator chamber 300.

As already suggested and with reference to Fig. 1, the docking station 400 of the modular incubator system 500 of the first aspect of the present invention comprises one or more docking ports 402 for receiving a housing 302 of one or more of the incubator chambers 300.

In respect of one or more docking ports 402 of the docking station 400, the docking port comprises an image capturing device 408 for capturing an image of the interior 306 of a modular incubator chamber 300, once that modular incubator chamber is being docked in that docking port 402.

Now, in order to make the shutter mechanism 344 shift its configuration between its closed configuration and its open configuration the modular incubator system is provided with a shutter actuator which, upon being provided with an electric signal thereto, is being configured to exert a force to a first shutter mechanism engagement means 346 in order to shift the configuration of that shutter mechanism 344 of the incubator chamber 300 between the open configuration and the closed configuration, and vice versa.

In some embodiments the shutter actuator is being arranged within the housing 302 of the modular incubator chamber 300.

However, in the following we refer to embodiments in which the shutter actuator is being arranged in or at the docking ports 402 of the docking station 400.

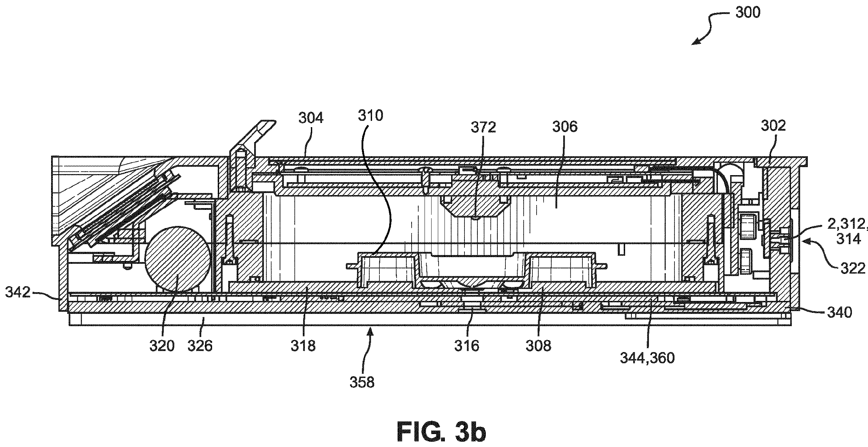

Fig. 3b is a cross-sectional view illustrating further details of the the modular incubator chamber of the present invention.

Fig. 3b shows that the housing 302 of the modular incubator chamber 300 comprises a transparent window 316 for allowing capturing of images of a biological material being accommodated in the interior thereof, through said transparent window. As seen the window is arranged at the bottom 358 of the housing 302 of the modular incubator chamber 300.

The modular incubator chamber 300 also comprises, in its interior 306, an electric heating element 318 for heating the interior of said modular incubator chamber. The modular incubator chamber also comprises a power source 320 in the form of a rechargeable battery for providing power to said heating element 318 which is being electrically connected to the power source 320. A light source 372 is attached to an inner side of the lid 304 of the modular incubator chamber 300.

As seen in Fig. 3b, the interior 306 of the modular incubator chamber 300 comprises a culture dish support 308 for positioning a culture dish 310. Hereby, one or more biological materials can be accommodated and incubated within the housing 302 of the modular incubator chamber 300.

Also seen in Fig. 3b is the chamber engagement means 326 which is being adapted to engage with docking port engagement means 414 of the docking port 402 into which the modular incubator chamber 300 is to be docked.

When such a proper positioning of the of the modular incubator chamber 300 in the docking port 402 has been attained via the chamber engagement means 326 of the chamber 300 and via the docking port engagement means 414 of the docking port 402, the relative position of the two electric connectors 410 and 322 of the docking port and the modular incubator chamber, respectively, will match pairwise so as to allow electric connection between the connectors 410 and 322.

We now return to Fig. 2a, 2b, 2c, 3a and 3b in order to further disclose features of the shutter mechanism itself.

Fig. 2a, 2b and 2c show that the housing 302 of the modular incubator chamber 300 comprises a transparent window 316 in the form of an elongate and linear opening extending in a direction Y which is perpendicular to the longitudinal direction X of the housing of the modular incubation chamber 300.

The shutter mechanism 344 comprises a plate shaped shutter element 360 which is displaceable along the longitudinal displacement direction D,X .

It is seen that the plate shaped shutter element 360 comprises an elongate opening 370 which is being aligned with the elongate shape of the transparent window 316 of the housing 302.

Designing the transparent window 316 of the housing 302 and the elongate opening 370 of the plate shaped shutter element 360 in an elongate shape allows for capturing images of a plurality of viable biological materials being accommodated in line in a culture dish which is being accommodated in the housing 302 of a modular incubator chamber 300.

Instead of having an elongate opening 370 in the plate shaped shutter element 360, one could alternatively let the rim of the plate shaped shutter element pass the transparent window 316 of the housing 302 in the shifting between the open and the closed configuration of the shutter mechanism 344. This is further described in the section below.

It is seen in fig. 2b that the plate shaped shutter element 360 is having a rim 367. In another embodiment than the one disclosed above (not shown in the figures), in its first position 362, said rim 367 of the plate shaped shutter element 360 of the shutter mechanism 344 is being positioned on one side in relation to said transparent window 316 of said housing 302 of said modular incubator chamber 300, thereby blocking transmittal of light, from outside said housing 302 of said modular incubator chamber 300 into the interior 306 of said housing through said transparent window 316 of said housing 302; and wherein in said second position 364, said rim 367 of said plate shaped shutter element 360 of the shutter mechanism 344 is being positioned on the opposite side of said transparent window 316 of said housing 302 of said modular incubator chamber 300, thereby enabling transmittal of light, from outside said housing 302 of said modular incubator chamber 300 into the interior 306 of said housing 306 through said transparent window 316 of said housing 302 and by passing said rim 367 of the plate shaped shutter element 360.

Accordingly, in such an embodiment the shifting of the shutter mechanism is simply performed by moving the rim 367 of the plate shaped shutter element 360 between two positions, one position is being on one side of the transparent window 316 of said housing 302, whereas the other position is on the opposite side of the transparent window 316 of the housing 302.

The first shutter mechanism engagement means 346 for enabling shifting the shutter mechanism between its open configuration and its closed configuration and vice versa, upon exerting a force thereto, comprises a first magnet 368 which is connected to the plate shaped shutter element 360 of said shutter mechanism 344.

The first magnet 368 is configured to be acted upon by a second magnet 450, thereby enabling the shifting of the configuration of the shutter mechanism 344 between said open configuration and said closed configuration, and vice versa, via movement of that second magnet 450.

In the embodiment illustrated in the figures, the second magnet 450 is arranged in the docking port 402 of the docking station as described below.

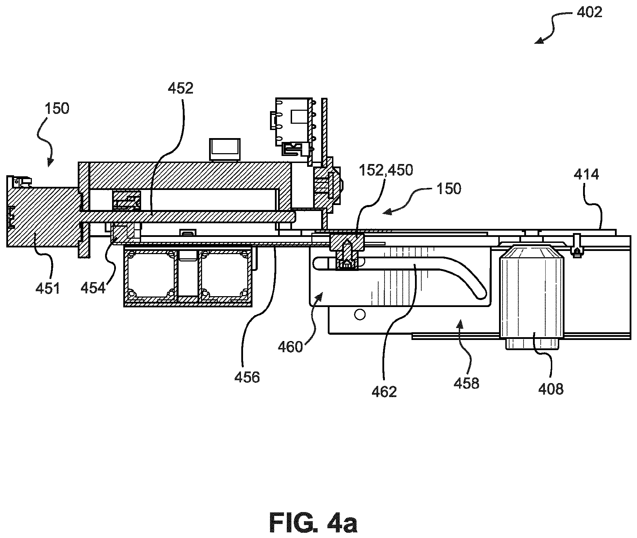

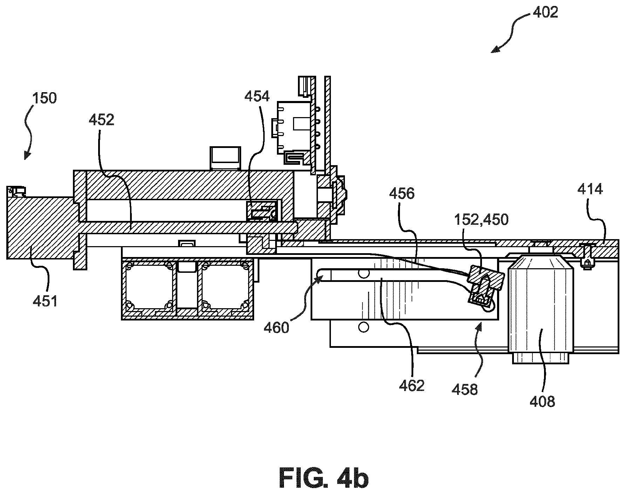

Fig. 4a and 4b are cross-sectional views illustrating details of a docking port of the docking system of the first aspect of the present invention.

Fig. 4a shows a single docking port 402 in a cross-sectional view. The docking port 402 comprises docking port engagement means 414 for entering into engagement with corresponding engagement means 326 of a modular incubator chamber 300.

The docking port 402 comprises image capturing means 408 for capturing images of a biological material which is being accommodated in the interior 306 of a modular incubator chamber 300 when being docked in that docking port 402.

Capturing of images requires that the shutter mechanism 344 of the modular incubator chamber 300 is being in its open configuration.

To this end, the docking port 402 comprises a shutter actuator 150.

The shutter actuator 150 comprises second shutter mechanism engagement means 152 for exerting a force to the first magnet 368 of the first shutter mechanism engagement means 346 in the form of the second magnet 450.

Accordingly, the second shutter mechanism engagement means 152 comprises a second magnet 450 which is configured for exerting a magnetic force to the first magnet 368 of the first shutter mechanism engagement means 346 upon movement of that second magnet 450, thereby enabling shifting the configuration of the shutter mechanism 344, between its closed configuration and its open configuration and vice versa.

The second magnet 450 is connected to the shutter actuator 150.

Fig. 4a shows that the shutter actuator 150 comprises an electromotor 451. The electromotor comprises a treaded rotatable axle 452. The shutter actuator 150 additionally comprises a treaded displacement element 454. The threads of the axle 452 is being in engagement with the treads of the displacement element 454.

It is seen in Fig. 4a that the threaded displacement element 454 is connected to the second magnet 450 by a connecting element 456.

This design thereby enables displacement of the second shutter mechanism engagement means 152 in the form of the second magnet 450 between a first extreme position 458 and a second extreme position 460, by rotation of said treaded rotatable axle 452 of said electromotor 451 in one rotational direction or in the opposite rotational direction.

In Fig. 4a the second magnet 450 is located near the second extreme position 460.

Fig. 4a also illustrates that the docking port 402 comprises a guide rail 462, and that the second shutter mechanism engagement means 152 (and second magnet 450) is beings slidable attached to the guide rail 462 and hence being configured to move along this guide rail when being moved between its first extreme position 458 and its second extreme position 460.

Fig. 4b shows the docking port 402 of Fig. 4a, however this time with the second magnet 450 in its first extreme position 458.

The movement of the second magnet 450 between its first extreme position 458 and its second extreme position 460 will, as further explained below, allow the plate shaped shutter element 360 of the modular incubator chamber 300 to shift its configuration between its open and its closed configuration, once that modular incubator chamber 300 is being docked in the docking port 402.

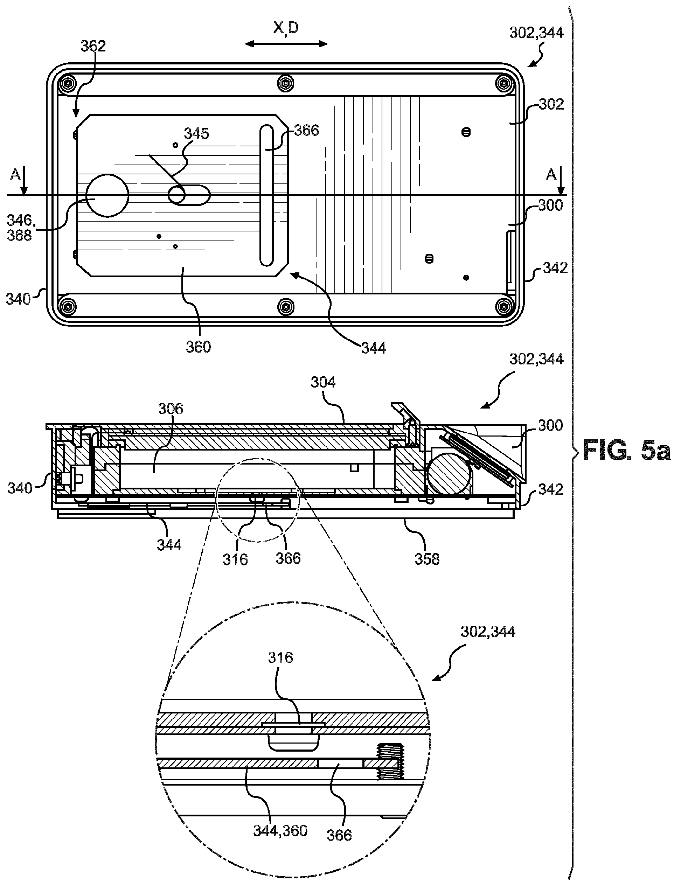

Fig. 5a illustrates the shutter mechanism of a modular incubator chamber in its closed configuration in a bottom plan view (top drawing of Fig. 5a), in a cross-sectional view (middle drawing of Fig. 5a), and in close-up cross-sectional view (bottom drawing of Fig. 5a).

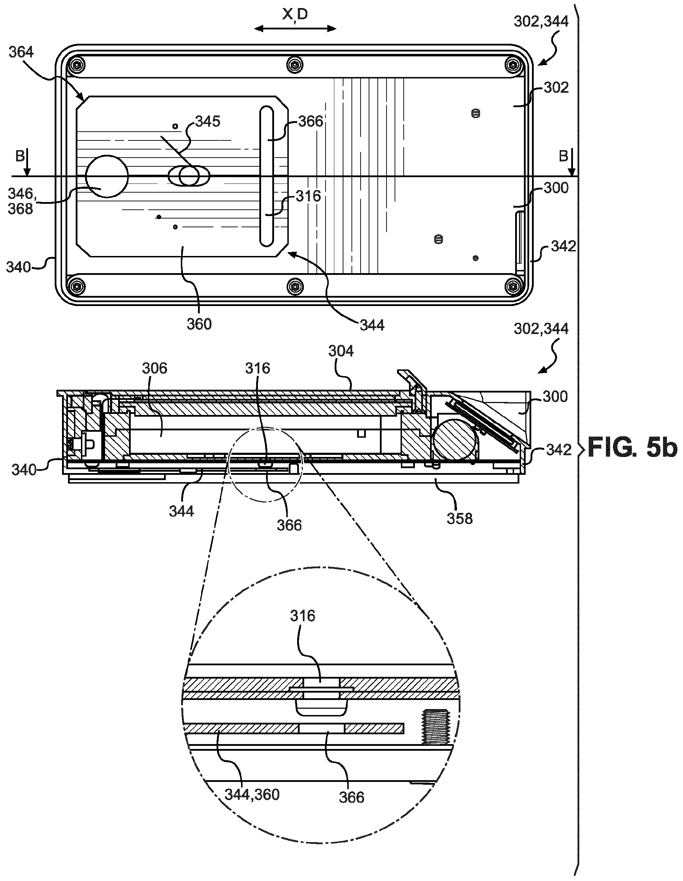

Fig. 5b illustrates the shutter mechanism of a modular incubator chamber in its open configuration in a bottom plan view (top drawing of Fig. 5b), in a cross-sectional view (middle drawing of Fig. 5b), and in close-up cross-sectional view (bottom drawing of Fig. 5b).

Top drawing of Fig. 5a is a bottom view illustrating the shutter mechanism 344 of the housing 302 of the modular incubator chamber 300. The shutter mechanism 344 comprises a plate shape shutter element 360 having a through-going opening 366 therein. The plate shape shutter element 360 is configured to be displaceable in a displacement direction D which is parallel to the longitudinal direction X of the housing 302 of the modular incubator chamber 300.

In Fig. 5a the plate shape shutter element 360 is arranged in its first position 362 in which the plate shaped element 360 is being displaced to the right, thereby being in its closed configuration.

The plate shape shutter element 360 comprises a first shutter mechanism engagement means 346 in the form of a first magnet 368. Upon exerting a magnetic force to this first magnet 368 it is possible to displace the plate shaped element 360 in a direction to the left so as to position the plate shaped element 360 in its second position 364, thereby being in its open configuration.

A spring 345 ensures that that when not acted upon by an external (magnetic) force, the plate shaped element 360 will stay in its first position in which the shutter 344 is in its closed configuration, thereby blocking image capturing and hence passage of light through the transparent window 316 of the housing 302 of the modular incubator chamber 300.

This is further illustrated in middle and bottom drawing of fig. 5a.

In these drawings we see that the through-going opening 366 of the plate shaped shutter element 360 is displaced relative to the transparent window 316 in the housing 302 of the modular incubator chamber. Thereby transmission of light from outside the housing 302 and into the interior 306 of the housing 302 of the modular incubator chamber 300 is blocked.

Fig. 5b shows the similar views as shown in Fig. 5a, however this time with the shutter mechanism 344 in its open configuration. This configuration is attained by having the plate shape shutter element 360 displaced to the left to its second position 364 (compare Fig. 5a and 5b).

It should be noted that as an alternative to make the first mechanism engagement means 346 act upon the second shutter mechanism engagement means 152 by means of a first magnet 368 and a second magnet 450 in order to shift the configuration of the shutter mechanism 344 between its open and closed configurations, the shutter mechanism 344 may comprise a first mechanism engagement means 346 in the form of a shutter lever which can be physically touched by the second shutter mechanism engagement means 152 in order to shift the configuration of the shutter mechanism 344 between its open and closed configurations.

Now returning to Fig. 4a and Fig. 4b it is easy to understand that once the modular incubator chamber 300 illustrated in Fig. 5a and 5b is being docked in the docking port 402 of the docking station 400, the actuation of the of the shutter actuator 150, that is the turning of the axle 452 of the electromotor 451, and the resulting movement of the second shutter mechanism engagement means 152 in the form of the second magnet 450 will be able to attract the first magnet 368 of the plate shaped shutter element 360 illustrated in Fig. 5a and 5b so as to displace this plate shaped shutter element 360 between its first position 362 and second position 364, thereby changing the configuration of the shutter mechanism 344 between its closed configuration and its open configuration, and vice versa.

In other words, once a modular incubator chamber 300 is being docked in the docking port 402, then when the second shutter mechanism engagement means 152 (second magnet 450) is moving in a direction from its first extreme position 458 to its second extreme position 460, the magnetic force between the first magnet 368 of the first shutter mechanism engagement means 346 and said second magnet 450 of the second shutter mechanism engagement means 152 is configured for bringing said shutter mechanism 344 into its open configuration; and when said second shutter mechanism engagement means 152 is moving in a direction from its second extreme position 460 to its first extreme position 458, then the magnetic force between said first magnet 368 of said first shutter mechanism engagement means 346 and said second magnet 450 of said second shutter mechanism engagement means 152 is configured for bringing said shutter mechanism into its closed configuration.

Fig. 4a and 4b show that part of guide rail 462 is having an essentially horizontally extension and that the guide rail 462, at a portion thereof corresponding to the first extreme position 458 of the second shutter mechanism engagement means 152 (second magnet 450), is inclined in a downward direction.

This downwardly inclined direction of the guide rail 462 ensures weakening the magnetic force between the first magnet 368 of the first shutter mechanism engagement means 346 and the second magnet 450 of the second shutter mechanism engagement means 152 in a situation where the shutter mechanism is in its closed configuration. Hereby a gentler attachment and detachment of the modular incubator chamber 300 into or from the docking port 402 of the docking station 400 is possible and this gentle attachment and detachment of the modular incubator chamber 300 into or from the docking port 402 will minimize physical impacts to the viable biological material caused by an otherwise sudden movement due to strong magnetic attraction which is difficult to fully counteract manually.

Accordingly, with the modular incubator system 500 of the present invention, viable biological materials can be incubated in one or more modular incubators 300 which are being docked in docking ports 402 of the docking station 400, and at the same time visual monitoring of the biological material can be conducted via the image capturing device 408. Moreover, the shutter mechanism 344 can be activated so as to block entrance of light into the interior 306 of the modular incubator chamber 300, except in these short moments in time where image capturing is being conducted, because in such situations the shutter mechanism 344 is brought into its open configuration, thereby enabling capturing of images through the transparent window 316 of the modular incubator chamber 300.

The present invention thereby allows for incubation of a biological material in the modular incubator chamber 300 and at the same time allows for visual monitoring of the morphological development of the biological material, while minimizing the detrimental effects involved in excessive exposure of the biological material to light.

It should be noted that in respect of a number N of adjacently arranged docking ports 402 of the docking station 400, these adjacently arranged docking ports 402 may share a common image capturing device 408 in the sense that one and only one image capturing device is responsible for capturing images relating to a modular incubator chamber 300 which is being docked in one of these N adjacently arranged docking ports 402.

In such a situation, a displacement device 482 in the form of a motorized suspension of the image capturing device 408 is being configured to be displaced, upon receiving a signal thereto, along a displacement track extending below these N number of adjacent docking ports 402 for enabling displacement of that common image capturing device 408 in relation to the N adjacently arranged docking ports 402 of the docking station 400. Thereby the common image capturing device 408 will be able to capture images of a biological material being accommodated in the interior 306 of a modular incubator chamber 300 being docked in any of said N docking ports 402 of the docking station 400.

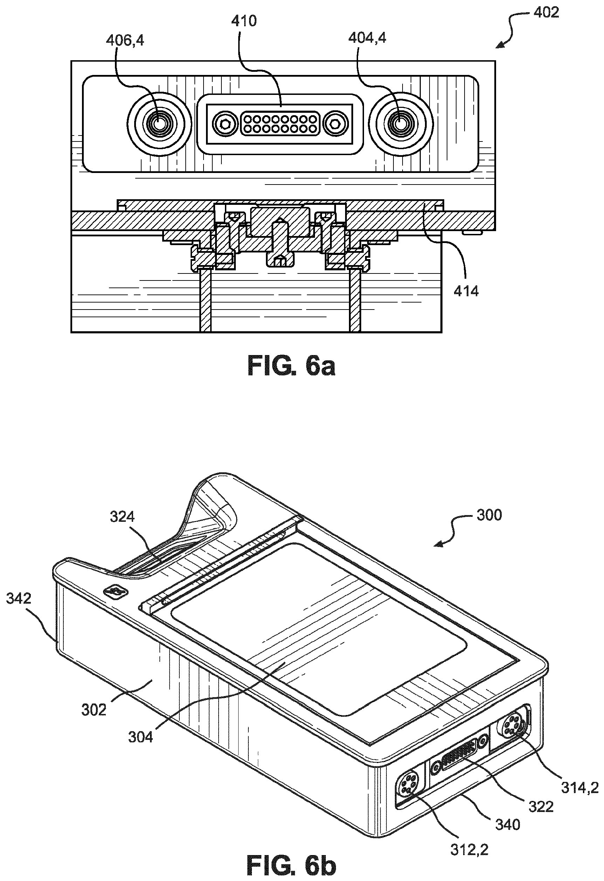

Fig. 6a is a front view looking into a docking port of a docking station of the modular incubator system of the present invention and Fig. 6b is a perspective view of a modular incubator chamber seen from the end which is to enter a docking port of the modular incubator system of the present invention.

Fig. 6a shows that the docking port 402 comprises electric connectors 410 which are configured to become into corresponding electric connectors 322 of a modular incubator chamber 300 as seen in Fig. 6b.

Fig. 6a also shows the that the docking port comprises a docking port outlet opening for gas 404 comprising a docking port valve 4 and a docking port inlet opening for gas 406 comprising a docking port valve 4. Likewise, Fig. 6b shows that the modular incubator chamber comprises a modular incubator chamber inlet opening for gas 312 with a modular incubator chamber valve 2, and a modular incubator chamber outlet opening for gas 314 with a modular incubator chamber valve 2.

It is seen in Fig. 6a and 6b that the docking port 402 comprises docking port engagement means 414 and that the modular incubator chamber 300 comprises corresponding incubator chamber engagement means 326.

When docking the modular incubator chamber 300 in the docking port 402 the incubation chamber engagement means 326 will enter into engagement with the docking port engagement means 414 so as to provide easy and proper positioning of the modular incubator chamber 300 in the docking port 402 of the docking station 400.

When such proper positioning of the of the modular incubator chamber 300 in the docking port 402 has been attained, the relative position of the two electric connectors 410 and 322, and the two inlet openings 312 and 406 with their respective valves 2,4 and the two outlet openings 314,404 with their respective valves 2,4 will match pairwise so as to allow electric connection between the connectors 410 and 322. Likewise, the gas openings 312, 314, 404 and 406 will pairwise match so that passage of gas from the docking port outlet opening for gas 404 into the interior 306 of the modular incubator chamber 300 via the modular incubator chamber inlet opening for gas 312 is enabled, and so that passage of gas from the interior 306 of the modular incubator chamber 300 is possible via the modular incubator chamber outlet opening for gas 314 and the the docking port inlet opening for gas 406.

Accordingly, the modular docking system 500 of the present invention allows for continuously providing gas into the interior 306 of the modular incubator chamber from a gas source 202.

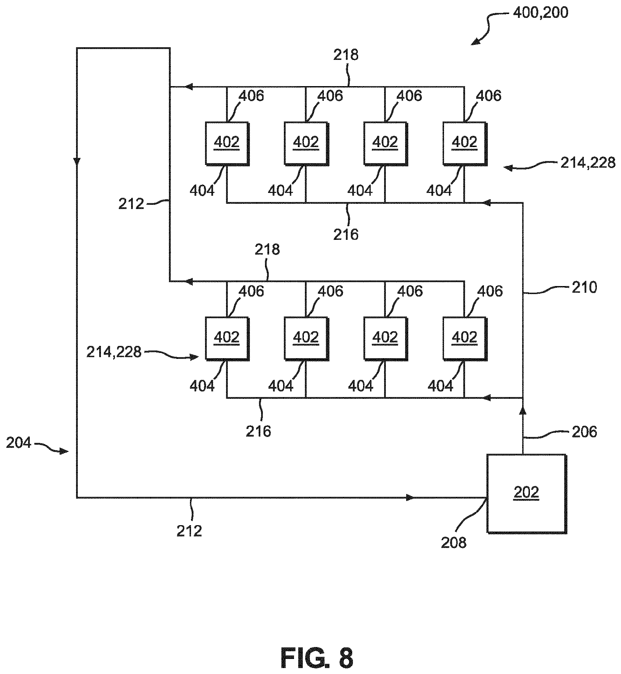

This is further illustrated with reference to Fig. 8,9 and 10.

However, first we turn to a more detailed description of a valve system to be used with the modular incubator system according to the first aspect of the present invention.

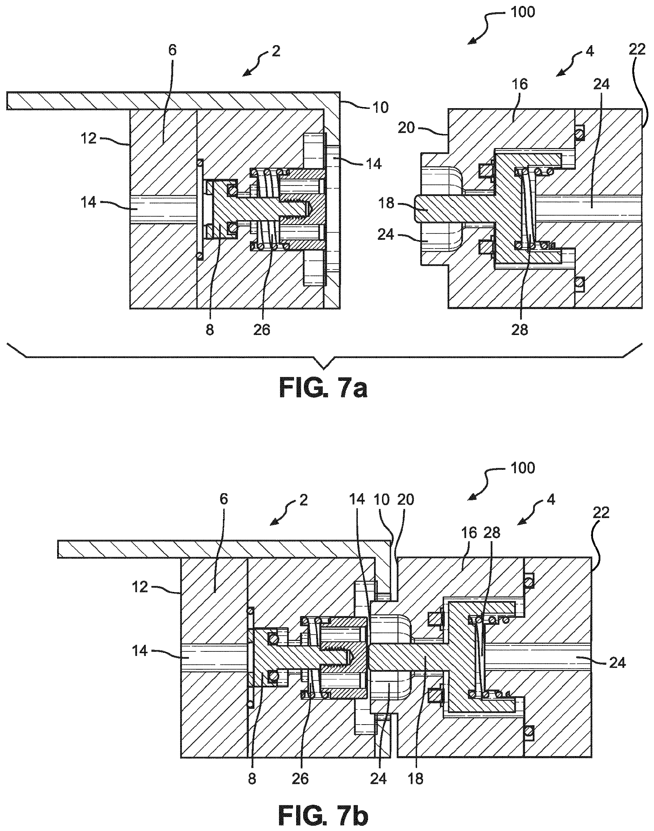

Fig. 7a and 7b illustrate the working modes of valves of a valve system to be used with the modular incubator chamber and the associated docking port of the docking station of the docking system of the present invention.

Fig. 7a is diagrammatic drawing illustrating a valve system 100 to be used with the modular incubator system of the present invention, where the two valves 2,4 of the valve system 100 are not engaged with each other, thereby attaining a closed configuration.

Fig. 7b is diagrammatic drawing illustrating the valve system 100 seen in Fig. 7a in which the two valves 2,4 of the valve system 100 are engaged with each other, thereby attaining an open configuration.

The valve 2 comprises a valve body 6 having a front end 10 and a rear end 12. A through-going channel 14 is arranged in the valve body 6 and a valve element 8 is being arranged in the through-going channel 14. The valve element 6 is spring-loaded by a spring 26.

The displaceable valve element 8 is being configured to be displaced in the through-going channel 14 of the valve body 6 by the spring 26 in such a way, that when not acted upon by an external force, the spring-loaded displaceable valve element 8 is being displaced in the through-going channel 14 of said valve body 6 by the spring 26 towards the front end 10 of the valve body 6. Thereby the valve 2 attains a closed configuration blocking passage of gas through the through-going channel 14.

This situation is illustrated in Fig. 7a.

By analogy, when acted upon by an external force, the spring-loaded displaceable valve element 8 is being displaced in the through-going channel 14 of said valve body 6 towards the rear end 12 of the valve body 6, thereby making the valve 2 attain an open configuration, allowing passage of gas through said through-going channel 14.

This situation is illustrated in Fig. 7b.

As to the valve 4, Fig. 7a shows that the valve 4 comprises a valve body 16 having a front end 20 and a rear end 22. A through-going channel 24 is arranged in the valve body 16 and a valve element 18 is being arranged in the through-going channel 24. The valve element 18 is spring-loaded by a spring 28.

The displaceable valve element 18 is being configured to be displaced in the through-going channel 24 of the valve body 16 by the spring 28 in such a way, that when not acted upon by an external force, the spring-loaded displaceable valve element 18 is being displaced in the through-going channel 24 of said valve body 16 by the spring 28 towards the first end 20 of the valve body 16. Thereby the valve 4 attains a closed configuration blocking passage of gas through the through-going channel 24.

This situation is illustrated in Fig. 7a.

By analogy, when acted upon by an external force, the spring-loaded displaceable valve element 18 is being displaced in the through-going channel 24 of said valve body 16 towards the rear end 22 of the valve body 16, thereby making the valve 4 attain an open configuration, allowing passage of gas through said through-going channel 24.

This situation is illustrated in Fig. 7b.

The valves 2 of the modular incubator chamber 300 and the valves 4 of the docking port 402 of the docking station are having dimensions and geometries in such a way that once docking the modular incubator chamber 300 in the docking port 402 of said docking station 400, the displaceable valve element 8 of the valve 2 and the displaceable valve element 18 of the valve 4 will displace each other into their respective valve bodies 6,16, thereby opening the valves 2,4 of said docking port outlet opening for gas 404 and said chamber inlet opening for gas 312; and also opening the valves 2,4 of the chamber outlet opening for gas 314 and the docking port inlet opening for gas 406.

Accordingly, using such valves 2,4 for the modular incubator chamber 300 and for the docking ports 402 of the docking station will automatically provide for opening the valves 2 of the modular incubator chamber 300 and the valves 4 of docking port 402, once that modular incubator chamber 300 is being docked in that docking port 402, thereby allowing passage of gas through the interior 306 of the modular incubator chamber 300, when being docking in the docking port 402 and also shutting off supply of gas into the docking port 402 and out of the modular incubator chamber 300, when that modular incubator chamber 300 is removed from the docking port.

It should be noted, that whereas the present description and the appended claims describe the modular incubator system 300 and the docking ports 402 in a way where the valves 2 are being arranged in the modular incubator system 300 and in a way where the valves 4 are being arranged in the docking ports 402, the opposite placement of the valves 2,4 is also possible.

In the above sections the general principle of a modular incubator system 500 comprising a docking station 400 with a plurality of docking ports 402 for receiving, by docking, a modular incubator chamber 300 has been described. In the sections below focus will be directed to features of supplying gas to the docking ports 402 of the docking station 400.

Fig. 8 is a diagram illustrating the concept of the gas supply system which may be incorporated in the docking station of the modular incubator system of the present invention.

Fig. 8 shows a gas supply system 200 to be used with a modular incubator system 500 according to the present invention. The gas supply system 200 comprises a gas source 202 and a gas distribution system 204.

The gas distribution system 204 comprises a plurality of docking ports 402 each having a docking port outlet opening for gas 404 and a docking port inlet opening for gas 406.

In respect of all the docking ports, the docking port outlet openings for gas 404 are in fluid connection with an inlet manifold 216 and the docking port inlet openings for gas 406 are in fluid connection with an outlet manifold 218.

A main gas supply line 210 supplies gas from a supply gas outlet 206 of the gas source 202 to the inlet manifolds 216, and a main gas return line 212 returns gas from the outlet manifolds 218 to a return gas inlet 208 of the gas source 202.

Thereby gas can be circulated from the gas source 202 via the gas distribution system 204 to the docking ports 402 and back to the gas source 202.

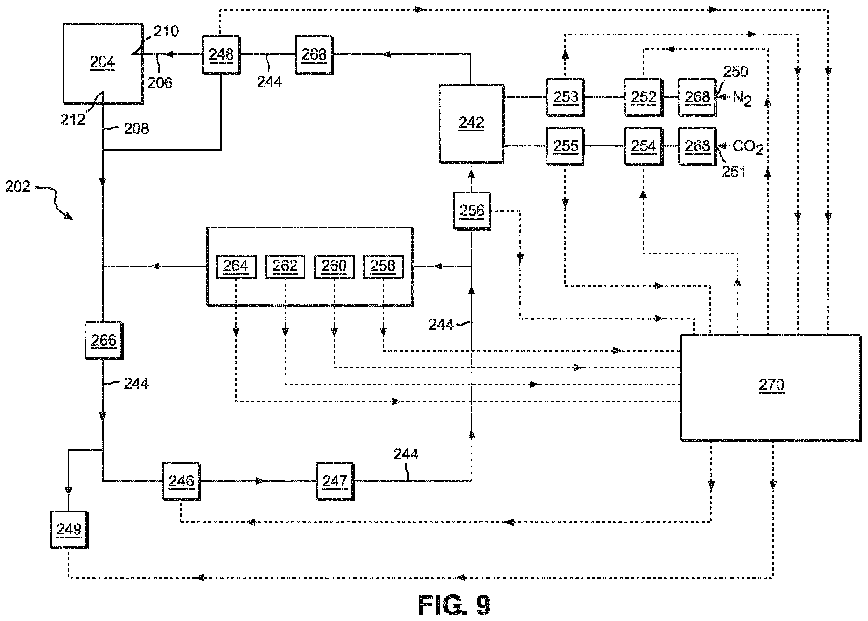

In order to secure a desired and predetermined and optimum gas composition of the gas supplied to the docking stations, the gas source is provided with specific features as disclosed with reference to figure 9.

Fig. 9 is a diagram illustrating one embodiment of a design of a gas supply system comprising a gas source to be used with the docking station of the modular incubator system of the present invention.

In fig. 9 solid lines represent flow lines for gas, whereas dashed lines represent signal lines for conveying electric signals or electric power.

Fig. 9 shows the gas distribution system 204 comprising its main gas supply line 210 and its main gas return line 212 (illustrated with the rectangle in upper left corner).

The main gas supply line 210 and the main gas return line 212 of the gas distribution system 204 is fluidly connected to a gas source 202 as described below.

The gas source 202 of said gas supply system 200 comprises a gas mixing box 242 connected to the supply gas outlet 206 and the return gas inlet 208 of the gas source.

The main gas supply line 210 of the gas distribution system 204 is being fluidly connected to the supply gas outlet 206, and the main gas return line 212 of the gas distribution system 204 is being fluidly connected to the return gas inlet 208 of the gas source 202.

Hereby a flow loop 244 comprising the gas distribution system 204 and the gas mixing box 242 is formed. The flow loop 244 comprises a pump 246 for circulating gas in that loop.

It is seen that the pump 246 is being arranged downstream in relation to the main gas return line 212. Also seen in Fig. 9 is that the flow loop 244 comprises a pump oscillation damper 247, which is being arranged immediately downstream in relation to the pump 246.

Moreover, the flow loop 244 comprises a pressure sensor 248 in the form of a differential pressure sensor for sensing the pressure of gas supplied to the main gas supply line 210, relative to the pressure in the return gas inlet line 208 of the gas distribution system 204. The pressure senor 248 is being arranged immediately upstream in relation to the main gas supply line 210 of the gas distribution system 204.

The flow loop 244 further comprises a release valve 249 for enabling pressure relief in the flow loop. The release valve is arranged immediately downstream in relation to the main gas return line 212 of the gas distribution system 402.

Also seen in Fig. 9 is that the gas mixing box 242 comprises an inlet for N₂ gas 250; and an inlet for CO₂ gas 251.

The inlet for N₂ gas 250 is fluidly connected to an N₂ valve 252 for regulating the inflow of N₂, and an N₂ mass flow sensor 253 arranged downstream of the N₂ valve 252 for sensing the amount of N₂ flowing into said gas mixing box 242.

The inlet for CO₂ gas 251 is fluidly connected to a CO₂ valve 254 for regulating the inflow of CO₂, and an CO₂ mass flow sensor 255 arranged downstream of the CO₂ valve 254 for sensing the amount of CO₂ flowing into the gas mixing box 242.

The flow loop 244 also comprises a mass flow sensor 256 arranged at an upstream position in relation to the gas mixing box 242 for sensing the amount of return gas entering the gas mixing box.

It is seen that the gas source 202 comprises an O₂ sensor 258 for sensing the concentration of O₂ exiting the gas distribution system 204; and that the gas source 202 comprises a CO₂ sensor 260 for sensing the concentration of CO₂ exiting the gas distribution system 204.

The O₂ sensor and the CO₂ sensor is arranged downstream in relation to the pump 246.

A temperature sensor 262 for sensing the temperature of gas circulating in said flow loop 244 is included in the gas source 202. The temperature sensor is arranged downstream in relation to the pump 246 at a position corresponding to the position of the O₂ sensor 258.

A pressure sensor 264 for sensing the absolute pressure in the flow loop 244 is included in the gas source 202. This pressure sensor is arranged downstream in relation to the pump 246, at a position corresponding to the position of the CO₂ sensor 260.

Also seen in Fig. 9 is that the flow loop 244 comprises a UV sanitizer 266 for sanitizing gas flowing in the flow loop 244 via electromagnetic radiation in the UV range. The UV sanitizer is arranged immediately downstream in relation to the main gas return line 212.

It is seen in Fig. 9 that the gas source 202 comprises filters 268 in the form of HEPA/VOCs filters. One such a filter is arranged immediately upstream in relation to the main gas supply line 210. Another such a filter is arranged immediately upstream in relation to the inlet for N₂ gas 250 into the gas mixing box 242; and a third such a filter is being arranged immediately upstream in relation to the inlet for CO₂ gas 251 into the gas mixing box 242.

Finally, it is seen in Fig. 9 that the gas source 202 comprises a gas mixing control system 270.

It is seen that the gas mixing control system 270 is electrically connected to one or more of the following sensors for receiving sensing signals therefrom: the N₂ mass flow sensor 253 for sensing the amount of N₂ flowing into the gas mixing box; the CO₂ mass flow sensor 255 for sensing the amount of CO₂ flowing into the gas mixing box; the mass flow sensor 256 for sensing the amount of return gas entering the gas mixing box; the O₂ sensor 258 for sensing the concentration of O₂ exiting the main gas return line 212 of the gas distribution system 204; the CO₂ sensor 260 for sensing the concentration of CO₂ exiting the main gas return line 212 of the gas distribution system 204; the temperature sensor 262 for sensing the temperature circulating in the flow loop 244; the pressure sensor 264 for sensing an absolute pressure in the flow loop 244, the pressure sensor 248 for sensing the pressure of gas supplied to the gas main gas supply line 210 of the distribution system 204.

Also seen in Fig. 9 is that the gas mixing control system 270 is electrically connected to one or more of the following elements for control thereof: the N₂ valve 252 for regulating the inflow of N₂ into the gas mixing box 242; the CO₂ valve 254 for regulating the inflow of CO₂ to the gas mixing box 242; the pump 246 for circulating gas in the flow loop 244; the release valve 249.

The control of the gas source by the gas mixing control system 270 is performed in accordance with two control regimes. The first control regime relates to controlling the pressure of gas exiting the supply gas outlet 206, and the second control regime relates to controlling the concentration of CO₂ and O₂ of gas exiting the supply gas outlet 206. The two control regimes are conducted concurrently. This is further explained below.

The gas mixing control system 270 is being configured to receive input from the pressure sensor 248 and on the basis thereof control the pump 246 and optionally also activate the release valve 249 in order to maintain a desired and predetermined pressure of gas supplied to the main gas supply line 210 of the gas distribution system 204.

The gas mixing control system 270 is further configured to receive input from the mass flow sensor 256, and on the basis on this input to determine the total amount of CO₂ gas and N₂ gas needed to be supplied via the inlet for CO₂ gas 251 and via the inlet for N₂ gas 250 according to desired and predetermined criteria.

Based on the information relating to the total amount of CO₂ gas and N₂ gas needed to be supplied, as described above, the gas mixing control system 270 will be able to determine the mutual proportion of the CO₂ gas and N₂ gas to be supplied to the gas mixing box 242.

This is performed by receiving input from the CO₂ sensor 260 and the O₂ sensor 258.

On the basis of the CO₂ concentration sensed, the gas mixing control system 270 will control the CO₂ valve 254, by transmitting a control signal thereto, and thereby regulate the inflow of CO₂ gas in order to reach a desired and predetermined CO₂ concentration.

Subsequently, the gas mixing control system 270 will on the basis of the O₂ concentration sensed, control the N₂ valve 252, by transmitting a control signal thereto, and thereby regulate the inflow of N₂ gas in order to reach a desired and predetermined O₂ concentration.

By using the gas source as disclosed above, a constant circulation of gas will be supplied to one or more modular incubator chamber 300 being docked in a respective docking port 402 of the docking station 400. By constantly regulating the inflow of CO₂ gas and N₂ gas based on sensed concentration of CO₂ and O₂ in the return gas from the gas distribution system 204, an optimum and predetermined gas composition can be maintained.

Due the design of the gas distribution system 204, an constant composition of gas flowing through each modular incubator chamber 300 can be upheld.

It should be noted that when reference is made to an upstream position relative to another position, that upstream position is construed to mean a position still within the gas source 202 and preferably not so much upstream that it passes the gas mixing box 242 or the gas distribution system 204.

Likewise, when reference is made to a downstream position relative to another position, that downstream position is construed to mean a position still within the gas source 202 and preferably not so much downstream that it passes the gas mixing box 242 or the gas distribution system 204.

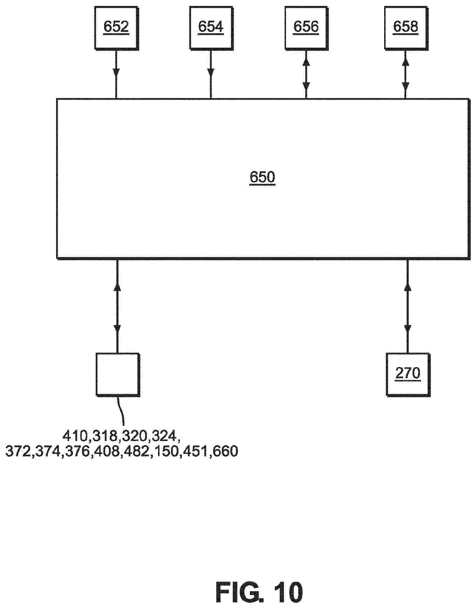

The modular incubator system 500 may comprise a control unit for controlling thereof. This is further illustrated with reference to Fig. 10.

Fig. 10 is a diagram illustrating the working mode of the controlling of the modular incubator system according to the invention.

Fig. 10 shows the control unit 650 for controlling the operation of the modular incubator system 500. The control unit is coupled to an input device 652 in the form of an alphanumerical input device for allowing a user to provide settings input relating to a desired operational protocol of said modular incubator system.

A display unit 654 for displaying, to a user, information relating to settings and/or operational status of one or more of the modular incubator chambers 300 is coupled to the control unit 654.

It is seen that the control unit 650 is coupled to a couple of electric connectors 410 of the docking ports 402 of the docking station 400. Thereby electrical power and electric signals can be provided to one or more modular incubator chambers 300 which is/are being docked into a docking port 402 of the docking station 400 of the modular incubator system 500.

By being connected to the docking ports 402 of the docking station 400 it will be possible, when one or more modular incubator chambers 300 is/are docked into a docking port 402 of the docking station 400, and by using the control unit 650, to control the following entities or parameters: the temperature in the interior 306 of said modular incubator chamber 300 by controlling said electric heating element 318, said thermostat (374 or said thermostatic circuit 376, provision of power to said electric power source 320; provision of signals to said display 324 of a modular incubator chamber 300 being docked in said docking port 402, switching on and off of said active light source 352 of a modular incubator chamber 300 being docked in said docking port 402, or adjusting light intensity emitted therefrom, said image capturing device 408 of a docking port 402, said displacement device 482 for displacing said image capturing device 408, said shutter actuator 150, optionally via said electromotor 451, said gas mixing control system 270; and said image processing unit 660.

The control unit 650 may comprises a CPU or other data processor 656 for processing the information involved in controlling the operation of the modular incubator system 500, e.g. by involving a computer program for handing the information involved in the controlling of the operation and the control unit 650 may also comprise a data storage 658.

Thereby automatic operation of the modular incubator system 500 may be performed in the sense that control unit 650 may independently and automatically, according to predetermined criteria, control one or more of the following: the temperature in the interior 306 of said modular incubator chamber 300 by controlling said electric heating element 318, said thermostat (374 or said thermostatic circuit 376, provision of power to said electric power source 320; provision of signals to said display 324 of a modular incubator chamber 300 being docked in said docking port 402, switching on and off of said active light source 352 of a modular incubator chamber 300 being docked in said docking port 402, or adjusting light intensity emitted therefrom, said image capturing device 408 of a docking port 402, said displacement device 482 for displacing said image capturing device 408, said shutter actuator 150, optionally via said electromotor 451, said gas mixing control system 270; and said image processing unit 660.

### List of reference numerals

- 2: Valve
- 4: Valve
- 6: First valve body of first valve
- 8: First valve element of first valve
- 10: Front end of first valve body
- 12: Rear end of first valve body
- 14: First throughgoing channel of first valve
- 16: Second valve body of second valve
- 18: Second valve element of second valve
- 20: Front end of second valve
- 22: Rear end of second valve
- 24: Second throughgoing channel of second valve
- 26: First spring of first valve
- 28: Second spring of second valve
- 150: Shutter actuator
- 152: Second shutter mechanism engagement means
- 200: Gas supply system
- 202: Gas source of gas supply system
- 204: Gas distribution system of gas supply system
- 206: Supply gas outlet of gas source
- 208: Return gas inlet of gas source
- 210: Main gas supply line of gas distribution system
- 212: Main gas return line of gas distribution system
- 214: Manifold pair
- 216: Inlet manifold of manifold pair
- 218: Outlet manifold of manifold pair
- 228: Group of docking ports
- 242: Gas mixing box
- 244: Flow loop of gas supply system
- 246: Pump of gas source
- 247: Pump oscillation damper
- 248: Pressure sensor for sensing pressure of gas supplied to main gas supply line
- 249: Release valve
- 250: Inlet for N₂ gas
- 251: Inlet for CO₂ gas
- 252: N₂ valve
- 253: N₂ mass flow sensor
- 254: CO₂ valve
- 255: CO₂ mass flow sensor
- 256: Mass flow sensor for sensing the amount of return gas flowing into gas mixing box
- 258: O₂ sensor
- 260: CO₂ sensor
- 262: Temperature sensor
- 264: Pressure sensor
- 266: UV sanitizer
- 268: Filter
- 270: Gas mixing control system
- 300: Modular incubator chamber
- 302: Housing of modular incubator chamber
- 304: Lid of modular incubator chamber
- 306: Interior of modular incubator chamber
- 308: Culture dish support
- 310: Culture dish
- 312: Modular incubator chamber inlet opening for gas
- 314: Modular incubator chamber outlet opening for gas
- 316: Transparent window of housing of modular incubator chamber
- 318: Electric heating element
- 320: Electric power source
- 322: Electric connectors of modular incubator chamber
- 324: Display of housing of modular incubator chamber
- 326: Incubation chamber engagement means of modular incubator chamber
- 340: First end of modular incubator chamber
- 342: Second end of modular incubator chamber
- 344: Shutter mechanism
- 345: Spring of shutter mechanism
- 346: First shutter mechanism engagement means
- 358: Bottom part of modular incubator chamber
- 360: Plate shape shutter element of shutter mechanism
- 362: First position of plate shape shutter element
- 364: Second position of plate shape shutter element
- 366: Through-going opening in plate shape shutter element
- 367: Rim of plate shaped shutter element
- 368: First magnet of first shutter mechanism engagement means
- 370: Elongate opening provided by shutter mechanism
- 372: Light source
- 374: Thermostat
- 376: Thermostatic circuit
- 400: Docking station
- 402: Docking port of docking station
- 404: Docking port outlet opening for gas
- 406: Docking port inlet opening for gas
- 408: Image capturing device of docking port of docking station
- 410: Electric connector of docking port
- 414: Docking port engagement means of docking port of docking station
- 450: Second magnet of second shutter mechanism engagement means
- 451: Electromotor
- 452: Threaded rotable axle of shutter actuator
- 454: Threaded displacement element
- 456: Connecting element
- 458: First extreme position of second shutter mechanism engagement means
- 460: Second extreme position of second shutter mechanism engagement means
- 462: Guide rail of docking port
- 482: Displacement device for displacing image capturing unit
- 500: Modular incubator system
- 650: Control unit
- 652: Input device
- 654: Display unit
- 656: Data processing unit
- 658: Data storage
- 660: Image processing unit
- D: Displacement direction of plate shaped shutter element
- X: Longitudinal direction of modular incubator chamber
- Y: Transversal direction perpendicular to longitudinal direction X

## Claims

1. A modular incubator system (500) for incubating a viable biological material M, said modular incubator system comprising:
- one or more modular incubator chambers (300) in combination with
- a docking station (400);
wherein in respect of one or more of said one or more modular incubator chambers (300), said modular incubator chamber (300) comprises a housing (302) having a first end (340) and a second end (342), thereby defining a longitudinal direction X between said first end and said second end;
wherein said housing comprises a lid (304), wherein said lid is being configured to be able to shift between an open configuration allowing access to the interior (306) of said modular incubator chamber and a closed configuration, sealing off access to the interior of said modular incubator chamber;
wherein said modular incubator chamber (300), at said interior (306) thereof, comprises a culture dish support (308) for positioning a culture dish (310) with the view to accommodate one or more biological materials M within the housing (302) of said modular incubator chamber (300);
wherein said housing (302) of said modular incubator chamber (300) comprises a transparent window (316) for enabling capturing of images of a biological material M being accommodated in the interior thereof, through said transparent window;
wherein said housing (302) of said modular incubator chamber (300) comprises a shutter mechanism (344), wherein said shutter mechanism is configured to be able to shift between an open configuration and a closed configuration and vice versa;
wherein said shutter mechanism (344) comprises a first shutter mechanism engagement means (346) for enabling shifting said shutter mechanism between its open configuration and its closed configuration and vice versa, upon exerting a force thereto;
wherein said shutter mechanism (344) is being arranged relative to said transparent window (316) of said housing in such a way that in its open configuration, said shutter mechanism (344) is enabling transmittal of light, from the outside, into the interior of said modular incubator chamber through said transparent window (316); and in such a way that in its closed configuration, said shutter mechanism (344) is blocking transmittal of light, from the outside, into the interior of said modular incubator chamber through said transparent window (316);
wherein said docking station (400) comprises one or more docking ports (402) for receiving a housing (302) of one or more of said incubator chambers (300);
wherein in respect of one or more docking ports (402) of said docking station (400), said docking port comprises an image capturing device (408) for capturing an image of the interior (306) of a modular incubator chamber (300), once being docked in said docking port (402).

2. A modular incubator system (500) according to claim 1, wherein in respect of one or more of said one or more modular incubator chambers (300) and in respect of one or more of said one or more docking ports (402) of said docking station (400), the position of said transparent window (316) of said modular incubator chamber (300) is adapted to the position of said image capturing device (408) in said docking port (402) in a way that enables capturing of images by said image capturing device (408) through said transparent window (316) of said modular incubator chamber (300), once said modular incubator chamber (300) is being docked in said docking port (402).

3. A modular incubator system (500) according to claim 1 or 2, wherein in respect of one or more of said modular incubator chambers (300) and/or in respect of one or more of said docking ports (402) of said docking station (400), said modular incubator system comprises a shutter actuator (150), such as an electric shutter actuator which, upon being provided with a signal thereto, such as an electric signal, is being configured to exert a force to said first shutter mechanism engagement means (346), in order to shift the configuration of said shutter mechanism (344) of said incubator chamber (300) between said open configuration and said closed configuration, and vice versa; optionally wherein in respect of one or more of said one or more modular incubator chambers (300), said shutter actuator (150) is being arranged in said modular incubator chamber (300) of said modular incubator system; or wherein in respect of one or more of said one or more docking ports (402) of said docking station (400), said shutter actuator (150) is being arranged in said docking port (402) of said modular incubator system.

4. A modular incubator system (500) according to any of the preceding claims, wherein in respect of one or more of said one or more modular incubator chambers (300), said modular incubator chamber (300), in the interior (306) thereof, comprises a light source (372) for directing light to the area of the culture dish support (308) of said modular incubator chamber (300), thereby enabling illumination of a viable biological material in a situation of capturing images of said viable biological material.

5. A modular incubator system (500) according to any of the preceding claims, wherein said modular incubator system (500) comprises an image processing unit (660) for image processing of images captured by said image capturing device (408), wherein said modular incubator system (400) optionally furthermore comprises a data storage (658) for storing images captured by said image capturing units (408) and/or for storing images processed by said image processing unit (660); optionally wherein one or more of said image capturing devices (408) of said docking ports (402) of said docking station is/are being coupled to an image processing unit (660).

6. A modular incubator system (500) according to any of the preceding claims, wherein in respect of one or more specific docking ports (402) of said docking station (400), said specific docking port comprises its own dedicated image capturing device (408) which is configured to only capture images relating to a modular incubator chamber (300) which is being docked in said specific docking port (402).

7. A modular incubator system (500) according to any of the preceding claims, wherein in respect of a number N of adjacently arranged docking ports (402) of said docking station (400), said adjacently arranged docking ports share a common image capturing device (408) in the sense that one and only one image capturing device is responsible for capturing images relating to a modular incubator chamber (300) which is being docked in one of said N adjacently arranged docking ports (402), wherein said docking station comprises a displacement device (482) for enabling displacement of said common image capturing device (408) in relation to said N adjacently arranged docking ports (402) of said docking station (400).

8. A modular incubator system (500) according to any of the preceding claims, wherein in respect of one or more of said modular incubator chambers (300), said modular incubator chamber comprises in its interior (306) an electric heating element (318) for heating the interior of said modular incubator chamber, and wherein said modular incubator chamber comprises a power source (320) for providing power to said heating element (318), wherein said electric heating element (318) is being electrically connected to said power source (320).

9. A modular incubator system (500) according to any of the preceding claims, wherein said modular incubator system (500) comprises a control unit (650) for controlling the operation of said modular incubator system (500);
optionally wherein said control unit (650) is being coupled to a data processing unit (656) and optionally also to a data storage (658) for aiding in handling information during controlling of said modular incubator system; and/or
optionally wherein said control unit (650) is being configured for enabling time lapse capturing of images by said image capturing devices (408).

10. A modular incubator chamber (300) comprising:
a housing (302) having a first end (340) and a second end (342), thereby defining a longitudinal direction X between said first end and said second end;
wherein said housing comprises a lid (304), wherein said lid is being configured to be able to shift between an open configuration allowing access to the interior (306) of said modular incubator chamber (300) and a closed configuration, sealing off access to the interior of said modular incubator chamber;
wherein said modular incubator chamber (300), at said interior (306) thereof, comprises a culture dish support (308) for positioning a culture dish (310) with the view to accommodate one or more biological materials M within the housing (302) of said modular incubator chamber (300);
wherein said housing (302) of said modular incubator chamber (300) comprises a transparent window (316) for enabling capturing of images of a biological material M being accommodated in the interior thereof, through said transparent window;
wherein said housing (302) of said modular incubator chamber (300) comprises a shutter mechanism (344), wherein said shutter mechanism is configured to be able to shift between an open configuration and a closed configuration and vice versa;
wherein said shutter mechanism (344) comprises a first shutter mechanism engagement means (346) for enabling shifting said shutter mechanism between its open configuration and its closed configuration and vice versa, upon exerting a force thereto;
wherein said shutter mechanism is being arranged relative to said transparent window (316) of said housing (302) in such a way that in its open configuration, said shutter mechanism (344) is enabling transmittal of light, from the outside, into the interior (306) of said modular incubator chamber (300) through said transparent window (316); and in such a way that in its closed configuration, said shutter mechanism (344) is blocking transmittal of light, from the outside, into the interior 306 of said modular incubator chamber (300) through said transparent window (316).

11. A modular incubator chamber (300) according to claim 10, wherein said incubator chamber (300) is comprising features as defined in respect of the modular incubator chamber (300) of the modular incubator system (500) according to any of the claims 1 - 9.

12. A docking station (400) for docking one or more modular incubator chambers (300); wherein said docking station comprises one or more docking ports (402) for receiving a housing (302) of one or more of said incubator chambers (300);
wherein in respect of one or more docking ports (402) of said docking station, said docking port comprises an image capturing device (408) for capturing an image of the interior (306) of a modular incubator chamber (300), once being docked in said docking port (402);
wherein one or more of said docking ports (402) comprises a shutter actuator (150), such as an electric shutter actuator which, upon being provided with a signal thereto, such as an electric signal, is being configured to exert a force to said first shutter mechanism engagement means (346) of an associated incubator chamber (300), in order to shift the configuration of said shutter mechanism (344) of said associated incubator chamber (300) between said open configuration and said closed configuration, and vice versa, when said incubator chamber (300) is being docked in said docking port (402).

13. A docking station (400) according to claim 12, wherein said docking station (400) is comprising features as defined in respect of the docking station (400) of the modular incubator system (500) according to any of the claims 1 - 9.

14. Use of a modular incubator system (500) according to any of the claims 1 - 9, or of a modular incubator chamber (300) according to any of the claims 10 or 11, or of a docking station (400) according to claim 13 for incubating a viable biological material; said biological material optionally is being an oocyte or an embryo, such as a human oocyte or a human embryo.

15. A method of incubating a viable biological material, wherein said method comprises:
i) providing a modular incubator system (500) according to any of the claims 1 - 9;
ii) providing a viable biological material;
iii) arranging said viable biological material in a culture dish (310) and subsequently arranging said culture dish in the interior (306) of a modular incubator chamber (300) of said modular incubator system (400);
iv) docking said modular incubator chamber (300) in a docking port (402) of said docking station (400) of said incubator system (500);
v) allowing said viable biological material to be incubated in said modular incubator chamber (300), while ensuring that said shutter mechanism (344) of said modular incubator chamber (300) is being in its closed configuration;
vi) whenever desired, shifting the configuration of said shutter mechanism (344) of said modular incubator chamber (300) to its open configuration and allowing said image capturing device (408) to capture one or more images of said biological material being accommodated in said culture dish (310);
vii) optionally, upon finishing capture of images of said biological material, shifting the configuration of said shutter mechanism (344) of said modular incubator chamber (300) to its closed configuration.

## Patentansprüche

1. Modulares Inkubatorsystem (500) zum Inkubieren eines lebensfähigen biologischen Materials M, wobei das modulare Inkubatorsystem Folgendes umfasst:
- eine oder mehrere modulare Inkubatorkammern (300) in Kombination mit
- einer Dockingstation (400);
wobei die modulare Inkubatorkammer (300) in Bezug auf eine oder mehrere von der einen oder den mehreren modularen Inkubatorkammern (300) ein Gehäuse (302) umfasst, das ein erstes Ende (340) und ein zweites Ende (342) aufweist, wodurch eine Längsrichtung X zwischen dem ersten Ende und dem zweiten Ende definiert ist;
wobei das Gehäuse einen Deckel (304) umfasst, wobei der Deckel konfiguriert ist, um in der Lage zu sein, zwischen einer offenen Konfiguration, die einen Zugang zum Inneren (306) der modularen Inkubatorkammer ermöglicht, und einer geschlossenen Konfiguration, die einen Zugang zum Inneren der modularen Inkubatorkammer verschließt, zu wechseln;
wobei die modulare Inkubatorkammer (300) in ihrem Inneren (306) eine Kulturschalenhalterung (308) zum Positionieren einer Kulturschale (310) mit der Absicht, ein oder mehrere biologische Materialien M innerhalb des Gehäuses (302) der modularen Inkubatorkammer (300) aufzunehmen, umfasst;
wobei das Gehäuse (302) der modularen Inkubatorkammer (300) ein transparentes Fenster (316) umfasst, um ein Erfassen von Bildern eines biologischen Materials M, das in seinem Inneren untergebracht ist, durch das transparente Fenster zu ermöglichen;
wobei das Gehäuse (302) der modularen Inkubatorkammer (300) einen Verschlussmechanismus (344) umfasst, wobei der Verschlussmechanismus konfiguriert ist, um in der Lage zu sein, zwischen einer offenen Konfiguration und einer geschlossenen Konfiguration und umgekehrt zu wechseln;
wobei der Verschlussmechanismus (344) eine erste Verschlussmechanismus-Eingriffseinrichtung (346) umfasst, um einen Wechsel des Verschlussmechanismus zwischen seiner offenen Konfiguration und seiner geschlossenen Konfiguration und umgekehrt zu ermöglichen, wenn eine Kraft darauf ausgeübt wird;
wobei der Verschlussmechanismus (344) in Bezug auf das transparente Fenster (316) des Gehäuses auf eine solche Weise angeordnet ist, dass der Verschlussmechanismus (344) in seiner offenen Konfiguration einen Durchgang von Licht von außen in das Innere der modularen Inkubatorkammer durch das transparente Fenster (316) ermöglicht; und auf eine solche Weise, dass der Verschlussmechanismus (344) in seiner geschlossenen Konfiguration einen Durchgang von Licht von außen in das Innere der modularen Inkubatorkammer durch das transparente Fenster (316) blockiert;
wobei die Dockingstation (400) eine oder mehrere Dockinganschlüsse (402) zum Aufnehmen eines Gehäuses (302) einer oder mehrerer der Inkubatorkammern (300) umfasst;
wobei der Dockinganschluss in Bezug auf eine oder mehrere Dockinganschlüsse (402) der Dockingstation (400) eine Bilderfassungsvorrichtung (408) zum Erfassen eines Bilds des Inneren (306) einer modularen Inkubatorkammer (300) umfasst, sobald sie in dem Dockinganschluss (402) angedockt ist.

2. Modulares Inkubatorsystem (500) nach Anspruch 1, wobei die Position des transparenten Fensters (316) der modularen Inkubatorkammer (300) in Bezug auf eine oder mehrere von der einen oder den mehreren modularen Inkubatorkammern (300) und in Bezug auf einen oder mehrere von dem einen oder den mehreren Dockinganschlüssen (402) der Dockingstation (400) an die Position der Bilderfassungsvorrichtung (408) in dem Dockinganschluss (402) auf eine Weise angepasst ist, die ein Erfassen von Bildern durch die Bilderfassungsvorrichtung (408) durch das transparente Fenster (316) der modularen Inkubatorkammer (300) ermöglicht, sobald die modulare Inkubatorkammer (300) in dem Dockinganschluss (402) angedockt ist.

3. Modulares Inkubatorsystem (500) nach Anspruch 1 oder 2, wobei das modulare Inkubatorsystem in Bezug auf eine oder mehrere der modularen Inkubatorkammern (300) und/oder in Bezug auf eine oder mehrere der Dockinganschlüsse (402) der Dockingstation (400) einen Verschlussaktuator (150) umfasst, wie einen elektrischen Verschlussaktuator, der, wenn ihm ein Signal, wie ein elektrisches Signal, bereitgestellt wird, konfiguriert ist, um eine Kraft auf die erste Verschlussmechanismus-Eingriffseinrichtung (346) auszuüben, um die Konfiguration des Verschlussmechanismus (344) der Inkubatorkammer (300) zwischen der offenen Konfiguration und der geschlossenen Konfiguration und umgekehrt zu wechseln; wobei optional der Verschlussaktuator (150) in Bezug auf eine oder mehrere von der einen oder den mehreren modularen Inkubatorkammern (300) in der modularen Inkubatorkammer (300) des modularen Inkubatorsystems angeordnet ist; oder wobei der Verschlussaktuator (150) in Bezug auf einen oder mehrere von dem einen oder den mehreren Dockinganschlüssen (402) der Dockingstation (400) in dem Dockinganschluss (402) des modularen Inkubatorsystems angeordnet ist.

4. Modulares Inkubatorsystem (500) nach einem der vorhergehenden Ansprüche, wobei die modulare Inkubatorkammer (300) in Bezug auf eine oder mehrere von der einen oder den mehreren modularen Inkubatorkammern (300) in ihrem Inneren (306) eine Lichtquelle (372) umfasst, um Licht auf den Bereich der Kulturschalenhalterung (308) der modularen Inkubatorkammer (300) zu richten, wodurch eine Beleuchtung eines lebensfähigen biologischen Materials in einer Situation eines Erfassens von Bildern des lebensfähigen biologischen Materials ermöglicht wird.

5. Modulares Inkubatorsystem (500) nach einem der vorhergehenden Ansprüche, wobei das modulare Inkubatorsystem (500) eine Bildverarbeitungseinheit (660) zum Bildverarbeiten von Bildern umfasst, die von der Bilderfassungsvorrichtung (408) erfasst werden, wobei das modulare Inkubatorsystem (400) optional ferner einen Datenspeicher (658) zum Speichern von Bildern, die von den Bilderfassungseinheiten (408) erfasst werden, und/oder zum Speichern von Bildern, die von der Bildverarbeitungseinheit (660) verarbeitet werden, umfasst; wobei optional eine oder mehrere der Bilderfassungsvorrichtungen (408) der Dockinganschlüsse (402) der Dockingstation mit einer Bildverarbeitungseinheit (660) gekoppelt ist/sind.

6. Modulares Inkubatorsystem (500) nach einem der vorhergehenden Ansprüche, wobei der spezifische Dockinganschluss in Bezug auf einen oder mehrere spezifische Dockinganschlüsse (402) der Dockingstation (400) seine eigene dedizierte Bilderfassungsvorrichtung (408) umfasst, die konfiguriert ist, um nur Bilder zu erfassen, die sich auf eine modulare Inkubatorkammer (300) beziehen, die in dem spezifischen Dockinganschluss (402) angedockt ist.

7. Modulares Inkubatorsystem (500) nach einem der vorhergehenden Ansprüche, wobei die benachbart angeordneten Dockinganschlüsse in Bezug auf eine Anzahl N von benachbart angeordneten Dockinganschlüssen (402) der Dockingstation (400) eine gemeinsame Bilderfassungsvorrichtung (408) in dem Sinne teilen, dass eine und nur eine Bilderfassungsvorrichtung für ein Erfassen von Bildern verantwortlich ist, die sich auf eine modulare Inkubatorkammer (300) beziehen, die in einer der N benachbart angeordneten Dockinganschlüsse (402) angedockt ist, wobei die Dockingstation eine Verschiebevorrichtung (482) umfasst, um eine Verschiebung der gemeinsamen Bilderfassungsvorrichtung (408) in Bezug auf die N benachbart angeordneten Dockinganschlüsse (402) der Dockingstation (400) zu ermöglichen.

8. Modulares Inkubatorsystem (500) nach einem der vorhergehenden Ansprüche, wobei die modulare Inkubatorkammer in Bezug auf eine oder mehrere der modularen Inkubatorkammern (300) in ihrem Inneren (306) ein elektrisches Heizelement (318) zum Beheizen des Inneren der modularen Inkubatorkammer umfasst, und wobei die modulare Inkubatorkammer eine Energiequelle (320) zum Bereitstellen von Energie an das Heizelement (318) umfasst, wobei das elektrische Heizelement (318) elektrisch mit der Energiequelle (320) verbunden ist.

9. Modulares Inkubatorsystem (500) nach einem der vorhergehenden Ansprüche, wobei das modulare Inkubatorsystem (500) eine Steuereinheit (650) zum Steuern des Betriebs des modularen Inkubatorsystems (500) umfasst;
wobei optional die Steuereinheit (650) mit einer Datenverarbeitungseinheit (656) und optional auch mit einem Datenspeicher (658) gekoppelt ist, um ein Handling von Informationen während eines Steuerns des modularen Inkubatorsystems zu unterstützen; und/oder
wobei optional die Steuereinheit (650) konfiguriert ist, um eine Zeitraffererfassung von Bildern durch die Bilderfassungsvorrichtungen (408) zu ermöglichen.

10. Modulare Inkubatorkammer (300), umfassend:
ein Gehäuse (302), das ein erstes Ende (340) und ein zweites Ende (342) aufweist, wodurch eine Längsrichtung X zwischen dem ersten Ende und dem zweiten Ende definiert ist;
wobei das Gehäuse einen Deckel (304) umfasst, wobei der Deckel konfiguriert ist, um in der Lage zu sein, zwischen einer offenen Konfiguration, die einen Zugang zum Inneren (306) der modularen Inkubatorkammer (300) ermöglicht, und einer geschlossenen Konfiguration, die einen Zugang zum Inneren der modularen Inkubatorkammer verschließt, zu wechseln;
wobei die modulare Inkubatorkammer (300) in ihrem Inneren (306) eine Kulturschalenhalterung (308) zum Positionieren einer Kulturschale (310) mit der Absicht, ein oder mehrere biologische Materialien M innerhalb des Gehäuses (302) der modularen Inkubatorkammer (300) aufzunehmen, umfasst;
wobei das Gehäuse (302) der modularen Inkubatorkammer (300) ein transparentes Fenster (316) umfasst, um ein Erfassen von Bildern eines biologischen Materials M, das in seinem Inneren untergebracht ist, durch das transparente Fenster zu ermöglichen;
wobei das Gehäuse (302) der modularen Inkubatorkammer (300) einen Verschlussmechanismus (344) umfasst, wobei der Verschlussmechanismus konfiguriert ist, um in der Lage zu sein, zwischen einer offenen Konfiguration und einer geschlossenen Konfiguration und umgekehrt zu wechseln;
wobei der Verschlussmechanismus (344) eine erste Verschlussmechanismus-Eingriffseinrichtung (346) umfasst, um einen Wechsel des Verschlussmechanismus zwischen seiner offenen Konfiguration und seiner geschlossenen Konfiguration und umgekehrt zu ermöglichen, wenn eine Kraft darauf ausgeübt wird;
wobei der Verschlussmechanismus in Bezug auf das transparente Fenster (316) des Gehäuses (302) auf eine solche Weise angeordnet ist, dass der Verschlussmechanismus (344) in seiner offenen Konfiguration einen Durchgang von Licht von außen in das Innere (306) der modularen Inkubatorkammer (300) durch das transparente Fenster (316) ermöglicht; und auf eine solche Weise, dass der Verschlussmechanismus (344) in seiner geschlossenen Konfiguration einen Durchgang von Licht von außen in das Innere (306) der modularen Inkubatorkammer (300) durch das transparente Fenster (316) blockiert.

11. Modulare Inkubatorkammer (300) nach Anspruch 10, wobei die Inkubatorkammer (300) Merkmale umfasst, wie sie in Bezug auf die modulare Inkubatorkammer (300) des modularen Inkubatorsystems (500) nach einem der Ansprüche 1 - 9 definiert sind.

12. Dockingstation (400) zum Andocken einer oder mehrerer modularer Inkubatorkammern (300); wobei die Dockingstation einen oder mehrere Dockinganschlüsse (402) zum Aufnehmen eines Gehäuses (302) einer oder mehrerer der Inkubatorkammern (300) umfasst;
wobei der Dockinganschluss in Bezug auf eine oder mehrere Dockinganschlüsse (402) der Dockingstation eine Bilderfassungsvorrichtung (408) zum Erfassen eines Bilds des Inneren (306) einer modularen Inkubatorkammer (300) umfasst, sobald sie in dem Dockinganschluss (402) angedockt ist;
wobei ein oder mehrere der Dockinganschlüsse (402) einen Verschlussaktuator (150) umfasst, wie einen elektrischen Verschlussaktuator, der, wenn ihm ein Signal, wie ein elektrisches Signal, bereitgestellt wird, konfiguriert ist, um eine Kraft auf die erste Verschlussmechanismus-Eingriffseinrichtung (346) einer assoziierten Inkubatorkammer (300) auszuüben, um die Konfiguration des Verschlussmechanismus (344) der assoziierten Inkubatorkammer (300) zwischen der offenen Konfiguration und der geschlossenen Konfiguration und umgekehrt zu wechseln, wenn die Inkubatorkammer (300) in dem Dockinganschluss (402) angedockt ist.

13. Dockingstation (400) nach Anspruch 12, wobei die Dockingstation (400) Merkmale umfasst, wie sie in Bezug auf die Dockingstation (400) des modularen Inkubatorsystems (500) nach einem der Ansprüche 1 - 9 definiert sind.

14. Verwendung eines modularen Inkubatorsystems (500) nach einem der Ansprüche 1 - 9 oder einer modularen Inkubatorkammer (300) nach einem der Ansprüche 10 oder 11 oder einer Dockingstation (400) nach Anspruch 13 zum Inkubieren eines lebensfähigen biologischen Materials; wobei das biologische Material optional eine Eizelle oder ein Embryo, wie eine menschliche Eizelle oder ein menschlicher Embryo, ist.

15. Verfahren zum Inkubieren eines lebensfähigen biologischen Materials, wobei das Verfahren Folgendes umfasst:
i) Bereitstellen eines modularen Inkubatorsystems (500) nach einem der Ansprüche 1 - 9;
ii) Bereitstellen eines lebensfähigen biologischen Materials;
iii) Anordnen des lebensfähigen biologischen Materials in einer Kulturschale (310) und anschließendes Anordnen der Kulturschale im Inneren (306) einer modularen Inkubatorkammer (300) des modularen Inkubatorsystems (400);
iv) Andocken der modularen Inkubatorkammer (300) in einem Dockinganschluss (402) der Dockingstation (400) des Inkubatorsystems (500);
v) Ermöglichen, dass das lebensfähige biologische Material in der modularen Inkubatorkammer (300) inkubiert wird, während sichergestellt ist, dass der Verschlussmechanismus (344) der modularen Inkubatorkammer (300) in seiner geschlossenen Konfiguration ist;
vi) wann immer gewünscht, Wechseln der Konfiguration des Verschlussmechanismus (344) der modularen Inkubatorkammer (300) in seine offene Konfiguration und Ermöglichen, dass die Bilderfassungsvorrichtung (408) ein oder mehrere Bilder des biologischen Materials, das in der Kulturschale (310) untergebracht ist, erfasst;
vii) optional, nach Beendigen eines Erfassens von Bildern des biologischen Materials, Wechseln der Konfiguration des Verschlussmechanismus (344) der modularen Inkubatorkammer (300) in seine geschlossene Konfiguration.

## Revendications

1. Système d'incubateur modulaire (500) destiné à incuber un matériau biologique viable M, ledit système d'incubateur modulaire comprenant :
- une ou plusieurs chambres d'incubateur modulaire (300) en combinaison avec
- une station d'accueil (400) ;
en ce qui concerne une ou plusieurs desdites une ou plusieurs chambres d'incubateur modulaire (300), ladite chambre d'incubateur modulaire (300) comprenant un boîtier (302) possédant une première extrémité (340) et une seconde extrémité (342), ce qui permet de définir une direction longitudinale X entre ladite première extrémité et ladite seconde extrémité ;
ledit boîtier comprenant un couvercle (304), ledit couvercle étant conçu pour pouvoir passer d'une configuration ouverte permettant l'accès à l'intérieur (306) de ladite chambre d'incubateur modulaire à une configuration fermée, fermant hermétiquement l'accès à l'intérieur de ladite chambre d'incubateur modulaire ;
ladite chambre d'incubateur modulaire (300), au niveau dudit intérieur (306) de celle-ci, comprenant un support de boîte de culture (308) destiné à positionner une boîte de culture (310) de manière à recevoir un ou plusieurs matériaux biologiques M à l'intérieur du boîtier (302) de ladite chambre d'incubateur modulaire (300) ;
ledit boîtier (302) de ladite chambre d'incubateur modulaire (300) comprenant une fenêtre transparente (316) destinée à permettre la capture d'images d'un matériau biologique M étant reçu à l'intérieur de celui-ci, à travers ladite fenêtre transparente ;
ledit boîtier (302) de ladite chambre d'incubateur modulaire (300) comprenant un mécanisme d'obturation (344), ledit mécanisme d'obturation étant conçu pour pouvoir passer d'une configuration ouverte à une configuration fermée et vice versa ;
ledit mécanisme d'obturation (344) comprenant un premier moyen d'engagement de mécanisme d'obturation (346) destiné à permettre le déplacement dudit mécanisme d'obturation entre sa configuration ouverte et sa configuration fermée et vice versa, en exerçant une force sur celui-ci ;
ledit mécanisme d'obturation (344) étant agencé par rapport à ladite fenêtre transparente (316) dudit boîtier de manière à ce que, dans sa configuration ouverte, ledit mécanisme d'obturation (344) permette la transmission de la lumière, de l'extérieur, à l'intérieur de ladite chambre d'incubateur modulaire à travers ladite fenêtre transparente (316) ; et de manière à ce que, dans sa configuration fermée, ledit mécanisme d'obturation (344) bloque la transmission de la lumière, de l'extérieur, à l'intérieur de ladite chambre d'incubateur modulaire à travers ladite fenêtre transparente (316) ;
ladite station d'accueil (400) comprenant un ou plusieurs ports d'accueil (402) destinés à recevoir un boîtier (302) d'une ou plusieurs desdites chambres d'incubateur (300) ;
en ce qui concerne un ou plusieurs ports d'accueil (402) de ladite station d'accueil (400), ledit port d'accueil comprenant un dispositif de capture d'images (408) destiné à capturer une image de l'intérieur (306) d'une chambre d'incubateur modulaire (300), une fois accueillie dans ledit port d'accueil (402).

2. Système d'incubateur modulaire (500) selon la revendication 1, en ce qui concerne une ou plusieurs desdites une ou plusieurs chambres d'incubateur modulaire (300) et en ce qui concerne un ou plusieurs desdits un ou plusieurs ports d'accueil (402) de ladite station d'accueil (400), ladite position de ladite fenêtre transparente (316) de ladite chambre d'incubateur modulaire (300) étant adaptée à la position dudit dispositif de capture d'images (408) dans ledit port d'accueil (402) d'une manière qui permet la capture d'images par ledit dispositif de capture d'image (408) à travers ladite fenêtre transparente (316) de ladite chambre d'incubateur modulaire (300), une fois que ladite chambre d'incubateur modulaire (300) est accueillie dans ledit port d'accueil (402).

3. Système d'incubateur modulaire (500) selon la revendication 1 ou 2, en ce qui concerne une ou plusieurs desdites chambres d'incubateur modulaire (300) et/ou en ce qui concerne un ou plusieurs desdits ports d'accueil (402) de ladite station d'accueil (400), ledit système d'incubateur modulaire comprenant un actionneur d'obturation (150), tel qu'un actionneur d'obturation électrique qui, lorsqu'il reçoit un signal, tel qu'un signal électrique, est conçu pour exercer une force sur ledit premier moyen d'engagement de mécanisme d'obturation (346), afin de faire passer la configuration dudit mécanisme d'obturation (344) de ladite chambre d'incubateur (300) de ladite configuration ouverte à ladite configuration fermée, et vice versa ; éventuellement, en ce qui concerne une ou plusieurs desdites une ou plusieurs chambres d'incubateur modulaire (300), ledit actionneur d'obturation (150) étant agencé dans ladite chambre d'incubateur modulaire (300) dudit système d'incubateur modulaire ; ou en ce qui concerne un ou plusieurs desdits un ou plusieurs ports d'accueil (402) de ladite station d'accueil (400), ledit actionneur d'obturation (150) étant agencé dans ledit port d'accueil (402) dudit système d'incubateur modulaire.

4. Système d'incubateur modulaire (500) selon l'une quelconque des revendications précédentes, en ce qui concerne une ou plusieurs desdites une ou plusieurs chambres d'incubateur modulaire (300), ladite chambre d'incubateur modulaire (300), à l'intérieur (306) de celle-ci, comprenant une source de lumière (372) destinée à diriger la lumière vers la zone du support de boîte de culture (308) de ladite chambre d'incubateur modulaire (300), ce qui permet l'éclairage d'un matériau biologique viable dans une situation de capture d'images dudit matériau biologique viable.

5. Système d'incubateur modulaire (500) selon l'une quelconque des revendications précédentes, ledit système d'incubateur modulaire (500) comprenant une unité de traitement d'images (660) destinée au traitement d'images des images capturées par ledit dispositif de capture d'images (408), ledit système d'incubateur modulaire (400) comprenant en outre éventuellement une mémoire de données (658) destinée à stocker des images capturées par lesdites unités de capture d'images (408) et/ou à stocker des images traitées par ladite unité de traitement d'images (660) ; éventuellement un ou plusieurs desdits dispositifs de capture d'images (408) desdits ports d'accueil (402) de ladite station d'accueil étant couplés à une unité de traitement d'images (660).

6. Système d'incubateur modulaire (500) selon l'une quelconque des revendications précédentes, en ce qui concerne un ou plusieurs ports d'accueil spécifiques (402) de ladite station d'accueil (400), ledit port d'accueil spécifique comprenant son propre dispositif de capture d'images dédié (408) qui est conçu pour capturer uniquement des images relatives à une chambre d'incubateur modulaire (300) qui est accueillie dans ledit port d'accueil spécifique (402).

7. Système d'incubateur modulaire (500) selon l'une quelconque des revendications précédentes, en ce qui concerne un nombre N de ports d'accueil agencés de manière adjacente (402) de ladite station d'accueil (400), lesdits ports d'accueil agencés de manière adjacente partageant un dispositif de capture d'images commun (408) en ce sens qu'un et un seul dispositif de capture d'images est responsable pour la capture d'images relatives à une chambre d'incubateur modulaire (300) qui est accueillie dans l'un desdits N ports d'accueil agencés de manière adjacente (402), ladite station d'accueil comprenant un dispositif de déplacement (482) destiné à permettre le déplacement dudit dispositif de capture d'images commun (408) par rapport auxdits N ports d'accueil agencés de manière adjacente (402) de ladite station d'accueil (400).

8. Système d'incubateur modulaire (500) selon l'une quelconque des revendications précédentes, en ce qui concerne une ou plusieurs desdites chambres d'incubateur modulaire (300), ladite chambre d'incubateur modulaire comprenant dans son l'intérieur (306) un élément de chauffage électrique (318) destiné à chauffer l'intérieur de ladite chambre d'incubateur modulaire, et ladite chambre d'incubateur modulaire comprenant une source d'énergie (320) destinée à fournir de l'énergie audit élément de chauffage (318), ledit élément de chauffage électrique (318) étant électriquement connecté à ladite source d'énergie (320).

9. Système d'incubateur modulaire (500) selon l'une quelconque des revendications précédentes, ledit système d'incubateur modulaire (500) comprenant une unité de commande (650) destinée à commander le fonctionnement dudit système d'incubateur modulaire (500) ;
éventuellement ladite unité de commande (650) étant couplée à une unité de traitement de données (656) et éventuellement également à une mémoire de données (658) pour aider à la manipulation des informations pendant la commande dudit système d'incubateur modulaire ; et/ou
éventuellement, ladite unité de commande (650) étant configurée pour permettre la capture d'images en laps de temps par lesdits dispositifs de capture d'images (408).

10. Chambre d'incubateur modulaire (300) comprenant :
un boîtier (302) possédant une première extrémité (340) et une seconde extrémité (342), ce qui permet de définir une direction longitudinale X entre ladite première extrémité et ladite seconde extrémité ;
ledit boîtier comprenant un couvercle (304), ledit couvercle étant conçu pour pouvoir passer d'une configuration ouverte permettant l'accès à l'intérieur (306) de ladite chambre d'incubateur modulaire (300) à une configuration fermée, fermant hermétiquement l'accès à l'intérieur de ladite chambre d'incubateur modulaire ;
ladite chambre d'incubateur modulaire (300), au niveau dudit intérieur (306) de celle-ci, comprenant un support de boîte de culture (308) destiné à positionner une boîte de culture (310) de manière à recevoir un ou plusieurs matériaux biologiques M à l'intérieur du boîtier (302) de ladite chambre d'incubateur modulaire (300) ;
ledit boîtier (302) de ladite chambre d'incubateur modulaire (300) comprenant une fenêtre transparente (316) destinée à permettre la capture d'images d'un matériau biologique M étant reçu à l'intérieur de celui-ci, à travers ladite fenêtre transparente ;
ledit boîtier (302) de ladite chambre d'incubateur modulaire (300) comprenant un mécanisme d'obturation (344), ledit mécanisme d'obturation étant conçu pour pouvoir passer d'une configuration ouverte à une configuration fermée et vice versa ;
ledit mécanisme d'obturation (344) comprenant un premier moyen d'engagement de mécanisme d'obturation (346) destiné à permettre le passage dudit mécanisme d'obturation de sa configuration ouverte à sa configuration fermée et vice versa, en exerçant une force sur celui-ci ;
ledit mécanisme d'obturation étant agencé par rapport à ladite fenêtre transparente (316) dudit boîtier (302) de manière à ce que, dans sa configuration ouverte, ledit mécanisme d'obturation (344) permette la transmission de la lumière, de l'extérieur, à l'intérieur (306) de ladite chambre d'incubateur modulaire (300) à travers ladite fenêtre transparente (316) ; et de manière à ce que, dans sa configuration fermée, ledit mécanisme d'obturation (344) bloque la transmission de la lumière, de l'extérieur, à l'intérieur (306) de ladite chambre d'incubateur modulaire (300) à travers ladite fenêtre transparente (316).

11. Chambre d'incubateur modulaire (300) selon la revendication 10, ladite chambre d'incubateur (300) comprenant des caractéristiques telles que définies en ce qui concerne la chambre d'incubateur modulaire (300) du système d'incubateur modulaire (500) selon l'une quelconque des revendications 1 à 9.

12. Station d'accueil (400) destinée à accueillir une ou plusieurs chambres d'incubateur modulaire (300) ; ladite station d'accueil comprenant un ou plusieurs ports d'accueil (402) destinés à recevoir un boîtier (302) d'une ou plusieurs desdites chambres d'incubateur (300) ;
en ce qui concerne un ou plusieurs ports d'accueil (402) de ladite station d'accueil, ledit port d'accueil comprenant un dispositif de capture d'images (408) destiné à capturer une image de l'intérieur (306) d'une chambre d'incubateur modulaire (300), une fois accueillie dans ledit port d'accueil (402) ;
un ou plusieurs desdits ports d'accueil (402) comprenant un actionneur d'obturation (150), tel qu'un actionneur d'obturation électrique qui, lorsqu'il reçoit un signal, tel qu'un signal électrique, est configuré pour exercer une force sur ledit premier moyen d'engagement de mécanisme d'obturation (346) d'une chambre d'incubateur associée (300), afin de faire passer la configuration dudit mécanisme d'obturation (344) de ladite chambre d'incubateur associée (300) de ladite configuration ouverte à ladite configuration fermée, et vice versa, lorsque ladite chambre d'incubateur (300) est accueillie dans ledit port d'accueil (402).

13. Station d'accueil (400) selon la revendication 12, ladite station d'accueil (400) comprenant des caractéristiques telles que définies en ce qui concerne la station d'accueil (400) du système d'incubateur modulaire (500) selon l'une quelconque des revendications 1 à 9.

14. Utilisation d'un système d'incubateur modulaire (500) selon l'une quelconque des revendications 1 à 9, ou d'une chambre d'incubateur modulaire (300) selon l'une quelconque des revendications 10 ou 11, ou d'une station d'accueil (400) selon la revendication 13 pour incuber un matériel biologique viable ; ledit matériel biologique étant éventuellement un ovocyte ou un embryon, tel qu'un ovocyte humain ou un embryon humain.

15. Méthode d'incubation d'un matériau biologique viable, ladite méthode comprenant :
i) la fourniture d'un système d'incubateur modulaire (500) selon l'une quelconque des revendications 1 à 9 ;
ii) la fourniture d'un matériau biologique viable ;
iii) l'agencement dudit matériau biologique viable dans une boîte de culture (310) et l'agencement ultérieur de ladite boîte de culture à l'intérieur (306) d'une chambre d'incubateur modulaire (300) dudit système d'incubateur modulaire (400) ;
iv) l'accueil de ladite chambre d'incubateur modulaire (300) dans un port d'accueil (402) de ladite station d'accueil (400) dudit système d'incubateur (500) ;
v) l'autorisation de l'incubation dudit matériau biologique viable dans ladite chambre d'incubateur modulaire (300), tout en veillant à ce que ledit mécanisme d'obturation (344) de ladite chambre d'incubateur modulaire (300) se trouve dans sa configuration fermée ;
vi) lorsque cela est souhaité, le passage de la configuration dudit mécanisme d'obturation (344) de ladite chambre d'incubateur modulaire (300) à sa configuration ouverte et l'autorisation audit dispositif de capture d'images (408) de capturer une ou plusieurs images dudit matériau biologique reçu dans ladite boîte de culture (310) ;
vii) éventuellement, à la fin de la capture d'images dudit matériau biologique, le passage de la configuration dudit mécanisme d'obturation (344) de ladite chambre d'incubateur modulaire (300) à sa configuration fermée.
